# EUROPEAN PATENT APPLICATION

(11) **EP 1 482 052 A1**
(43) Date of publication of application: **01.12.2004**
(21) Application number: 03011184.3
(22) Date of filing: 27.05.2003
(51) Int. Cl.: C12N 15/86, C12N 15/62, C07K 19/00, C07K 16/28, C07K 16/46, C07K 14/705, A61K 38/17, A61K 35/76

(54) **Modified polypeptides for targeting cell-entry of the adenoviruses of subtype B**

(71) Applicant: Cytos Biotechnology AG, 8952 Schlieren (CH)
(72) Inventor: Beerli, Roger R., 8106 Adlikon b. Regensdorf (CH); Bachmann, Martin F., 8472 Seuzach (CH)
(74) Representative: Wichmann, Hendrik, Dr.

(57) **Abstract**

This invention relates to modified polypeptides comprising two functional components: first, a polypeptide derived from the extracellular region of CD46 as a specific binding site for adenoviruses of the subgroup B, and second, a component capable of binding to a cell surface molecule. Such modified polypeptides are able to direct adenovirus-infection specifically to cells having said cell surface molecule on their surface. The invention relates to nucleic acid sequences encoding fusion proteins comprising a) a polypeptide derived from the extracellular domain of CD46 and b) a heterologous polypeptide, methods for the production of the modified polypeptides and suitable recombinant expression vectors and host cells. Pharmaceutical compositions comprising the modified polypeptide of the invention are useful together with recombinant, genetically engineered adenovirus of subtype B for the treatment and prophylaxis of disorders and diseases, like cancer.

## Description

### BACKGROUND OF THE INVENTION

Adenoviruses are non-enveloped DNA viruses with a broad host spectrum. They have been detected in numerous animal species and can infect various cell types, although with varying efficiency. In humans, adenoviruses are common pathogens and a major cause of respiratory and gastrointestinal infections (Horwitz, MS. (1996) Adenoviruses. In *Virology*, *3*^{*rd*} *edition* (editors B. N. Fields, D. M. Knipe, and P. M. Howley), 2149-2171. Lippincott Williams & Wilkins), as well as infections of the heart (Martin AB, et al. (1994) *Circulation* **90**, 330-339). Numerous serotypes have been characterized within each species which exhibit a genomic organization and an infectious cycle which are comparable (Shenk T. (1996) Adenoviridae: the viruses and their replication. In *Virology, 3*^{*rd*} *edition* (editors B. N. Fields, D. M. Knipe, and P. M. Howley), 2111-2148. Lippincott Williams & Wilkins). In general, the adenoviral genome consists of a double-stranded linear DNA molecule of about 36 kb containing the genes encoding the viral proteins and, at its ends, two inverted repeats which are involved in replication, and the encapsidation region (Shenk, supra).

Viruses infect target cells by attachment to specific cell surface receptors, and the tissue distribution of receptor molecules is therefore an important determinant of virus tropism. In the case of adenoviruses, cell attachment is mediated by elongated fibers protruding from each of the 12 vertices of the icosahedral viral capsid (Shenk, supra). The fact that isolated fibers bind cells with high affinity and block virus attachment and infection, clearly demonstrates that fiber attachment to a cell surface receptor is a critical step during infection (Defer C, et al. (1990) *J Virol.* **64**, 3661-73; Wickham TJ, et al. (1993) *Cell* **73**, 309-19).

Adenoviruses engineered to carry therapeutic genes have a tremendous potential as vectors for gene therapy. The characterization of the molecular mechanism of virus cell attachment and entry is therefore of prime importance, not only for the understanding of virus tropism, but also for the development of agents that modulate virus binding or alter the specificity of binding. Adenoviruses of the subgroup C, including Ad2 and Ad5, as well as most other subgroups, utilize the coxsackie-adenovirus receptor (CAR) protein as their primary receptor (Mayr GA, and Freimuth P. (1997) *J Virol.* **71**, 412-8). Competition studies demonstrated that adenoviruses of the subgroup B, such as Ad3 and Ad7, infect cells via binding to a different and hitherto unidentifed receptor (Defer et al., supra; Stevenson SC, et al. (1995) *J. Virol.* **69**, 2850-2857).

In the case of cancer gene therapy, it has been observed that many tumor types are poorly infected by Ad5, due to low levels of CAR expression. It has been shown that this can be circumvented by the production of CAR-independent virus, in which the Ad5 knob is replaced by the knob region of Ad3 (Kawakami Y, et al. (2003) *Cancer Res* **63**, 1262-1269). Thus, while Ad5 is still the predominant vector used for gene therapy, there is increasing interest in the use of other serotypes including Ad3. The identification of the Ad3 receptor, used by adenoviruses of the subgroup B, would be of significant interest in the gene therapy field.

We now report the identification of CD46 as a receptor used by adenoviruses of the subgroup B, such as Ad3 and Ad7, for their cell entry.

### SUMMARY OF THE INVENTION

This invention relates to modified polypeptides comprising two functional components: first, a polypeptide derived from the extracellular region of CD46, also known as membrane cofactor protein (MCP), as a specific binding site for adenoviruses of the subgroup B (*e.g*. Ad3), and second, a component capable of binding to a cell surface molecule. Such bifunctional, modified polypeptides are able to direct adenovirus-infection specifically to such cells, which have said cell surface molecule on their surface. If the component capable of binding to a cell surface molecule is a polypeptide and the fusion between the two polypeptide components is a genetic linkage so as to form a fusion polypeptide, then the second component is not a polypeptide derived from CD55. Another aspect of the invention relates to nucleic acid molecules comprising a nucleotide sequence encoding a fusion protein comprising a) a polypeptide derived from the extracellular domain of CD46 and b) a heterologous polypeptide, as long as it is not derived from CD55. Another aspect of the invention relates to recombinant expression vectors and host cells comprising the nucleic acid molecules of the invention. In another aspect of the invention a pharmaceutical composition comprising a modified polypeptide of the invention is provided. In another aspect a method for the production of the modified polypeptides of the invention is provided. The modified polypeptides of the invention are useful in methods of treatment when administered together with recombinant, genetically engineered adenovirus of subtype B, and can be used in methods of preparing a medicament together with such adenoviruses, wherein the medicament can then be used, e.g. for the treatment and prophylaxis of cancer.

### DEFINITIONS

A "polypeptide" as used herein is a molecule comprising more than 20, in particular more than 25, 30, 40, 50, 60 and most preferably more than 70 amino acids, but less than 10000, in particular less than 9000, 8000, 7000, 6000, 5000, 4000, 3000 or 2000, most preferably less than 1500 amino acids. Also, polypeptides with substantial amino acid sequence identity and polypeptides, which contain a low percentage, e.g. less than 5%, 3% or even only up to 1%, of modified or non-natural amino acids are encompassed.

A "peptide" as used herein is a molecule comprising less than 20 amino acids, but preferably more than 4, 5, 6, 7, 8 or 9 amino acids.

A "modified" polypeptide is a polypeptide, which is not encoded as such by the genome of a naturally occurring species, in particular a polypeptide that is not identical to one of those polypeptides of the Genbank database as of May 26, 2003, with a naturally occurring species identified as their source. This means that a "modified" polypeptide does not occur as such in nature, but can be, and in particular was, produced by laboratory manipulations, such as genetic engineering techniques or chemical coupling of another molecule to a polypeptide. This term in particular encompasses all mutant polypeptides, in particular deletions, truncations, multiple substitutions and fusion polypeptides, which at one stage were produced by genetic engineering techniques. The term "modified" polypeptide as used herein preferably does not include a fusion polypeptide of the extracellular domain of CD46 with a polypeptide derived from CD55, preferably also not fusion polypeptides of the extracellular domain of CD46 with a component (b), which is a "tag" commonly used for facilitated protein purification, like a multi-Histidine-tag or a GST-tag, or which is a polypeptide commonly used in so-called two-hybrid assays, like a Gal4-domain. However, if the function of a component (b) is - in the context of a fusion polypeptide - provided by another polypeptide, like, e.g., a cell surface specific antibody or antibody fragment, then a "tag" useful for detection or purification may be present in the overall fusion polypeptide.

A polypeptide is derived from the extracellular domain of CD46, if it shows at least 50% identity with any 60 amino acids long fragment of the extracellular domain of human CD46, which is specified here as amino acids 35 to 328 of SEQ ID NO:2, preferably with any 75 amino acids, 90 amino acids or even 120 amino acids of the extracellular domain of human CD46. Preferably, also the sequence identity is higher for these stretches, and can be at least 70%, 75%, 80%, 85%, 90%, 95% or even 100%.

The overall structure of full length, wildtype human CD 46 is as follows: a N-terminal signal peptide of 34 amino acids is cleaved from the 384 amino acid precursor to form the mature 342-amino acid protein during membrane translocation. The mature protein has its N-terminus extending to the extracellular region, amino acids 329-351 (amino acid positions of CD46 are numbered in the context of the precursor protein) form a transmembrane domain and its C-terminus extends to the cytoplasm. The extracellular region of CD46 is thus formed by amino acids 35 to 328 of the precursor protein. This region has four cysteine-rich repeats (short consensus repeats, SCRs) of about 60 amino acids each and these repeats are similar to a consensus sequence found for repeats of several complement regulatory proteins. These SCRs are termed SCR1-4, with SCR1 extending from AA 35 to 95, SCR2 from 98 to 158, SCR3 from 161 to 224 and SCR4 from 227 to 284. C-terminal of the SCRs is a serin/threonine/proline-rich region (STP), which can be subdivided into STP-B from AA286 to 300 and STP-C, from AA 301 to 314. Some splicing variants of CD46 have an additional STP-A region with the sequence VLPPSSTKPPALSHS, SEQ ID NO: 3, inserted between SCR4 and STP-B of the reference wildtype CD46 polypeptide sequence. It is to be understood that polypeptides derived from extracellular domains of the CD46 splice variants, e.g. those described in Russell et al. (1992) Eur. J. Immunol. 22:1513-1518 or Purcell et al. (1991) Immunogenetics 33:335-344, or those splice variants described in the SWISSPROT entry P15529, may also be used as a polypeptide (a) of a modified polypeptide of the invention.

The term "at least one" as used here means "one and more than one", particularly one, two, three, four and five.

The term "at least X% identity with Y" as used herein means that if a given nucleic acid or polypeptide sequence is aligned to a nucleic acid sequence Y or a polypeptide sequence Y, respectively, X% of the bases or amino acids within the aligned region are identical. X can be at least 70, at least 75, at least 80, at least 85, at least 90 or at least 95. If polypeptide sequences are aligned, then the closely related residues aspartic acid and glutamic acid are usually regarded as being identical, as well as the closely related residues asparagine and glutamine. However, preferably the residues asparagine and glutamine are not regarded as being identical and more preferably also the residues aspartic acid and glutamic acid are also not regarded as being identical.

The term "antibody", as used herein refers to an immunological binding agent, including polyclonal and monoclonal antibodies. Depending on the type of constant domain in the heavy chains, antibodies are assigned to one of five major classes: IgA, IgD, IgE, IgG, and IgM. Several of these are further divided into subclasses or isotypes, such as IgG1, IgG2, IgG3, IgG4. The heavy-chain constant domains that correspond to the different classes of immunoglobulins are termed alpha, delta, epsilon, gamma and mu, respectively. The subunit structures and three-dimensional configurations of different classes of immunoglobulins are well known.

Antibodies may be also selected from modified antibodies, for example chemically or recombinantly produced antibodies or humanized antibodies.

The term "antibody fragment" is used to refer to any fragment of an antibody-like molecule that has an antigen binding region, and this term includes antibody fragments such as scFv, dsFv, scFab, Fab', Fab, F(ab')2, Fv, single domain antibodies (DABs), diabodies, and the like. The techniques for preparing and using various antibody-based constructs and fragments are well known in the art (see Kabat et al. (1991) J. Immunol. 147, 1709-19), specifically incorporated herein by reference.

"scFv" antibody fragments comprise the VH and VL domains of an antibody, wherein these domains are present in a single polypeptide chain. Generally, the scFv polypeptide further comprises a polypeptide linker between the VH and VL domains that enables the scFv to form the desired structure for antigen binding.

A "Fv" fragment is the smallest antibody fragment that retains an intact antigen binding site.

A "dsFv" is a disulfide stabilized Fv.

A "Fab" fragment, is an antigen binding fragment, containing complete light chains paired with the VH and CH1 domains of the heavy chain.

A "Fab"' fragment, is a reduced F(ab')2 fragment.

A "F(ab')2" fragment, is a bivalent fragment comprising two Fab fragments linked by a disulfide bridge at the hinge region.

A "single domain antibody (DAB)" is an antibody with only one (instead of two) protein chain derived from only one of the domains of the antibody structure. Dabs exploit the finding that, for some antibodies, half of the antibody molecule binds to its target antigen almost as well as the whole molecule (Davies et al. (1996) Protein Eng. 9: 531-537.

"Diabodies" are bivalent or bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (Holliger et al. (1993) Proc. Natl. Acad. Sci. USA, 90, 6444-6448).

A "component binding specifically to a cell surface molecule" as mentioned herein can be a small organic molecule, a peptide or a polypeptide which binds to a cell surface molecule under the buffer conditions given in Example 2. The dissociation constant between said component and the cell surface molecule can be measured, e.g. by use of the so-called BIACORE System (see, for example, Fivash et al. Curr Opin Biotechnol. (1998) 9, 97-101) and "binding specifically" can then be understood to mean that the dissociation constant between the component and the cell surface molecule is lower than 10 µM, preferably lower than 1 µM, more preferably lower than 500, 400, 300, 200, 100, 50, 20 nM, most preferably from 0,1 nM to 20 nM if measured under standard conditions, for example at 20°C, ambient pressure and in a suitable buffer, e.g. 20 mM Tris, 100 mM NaCl, 0,1 mM EDTA at an overall pH of 7.0. Preferably, the dissociation constant between the component and the extracellular region of a plasma protein, like human serum albumin or bovine serum albumin, is higher than 100 µM, preferably higher than 1 mM, or is alternatively at least 50-fold, preferably at least 200-fold, 1000-fold or 5000-fold worse (higher) than the dissociation constant between the component and the cell surface molecule to which it binds specifically.

A "small organic chemical molecule" as used herein is a molecule comprising at least one carbon atom and at least one hydrogen atom. Its molecular weight is between 30D and 5kD, preferably from 100D to 2kD.

A "polypeptide of the complement pathway" as used herein is a polypeptide of the classical pathway, the mannan-binding lectin pathway or the alternative pathway, and can, in particular, also be a polypeptide involved in the regulation of one of these pathways. Such complement-regulatory proteins are, in particular, C1NIH, C4BP, CR1, DAF, H, I, P and CD59.

A "non-proteinaceous hormone" is a hormone not consisting of linearly linked amino acid units. Examples of such hormones are cyclic peptide hormones, such as somatostatin and urotensin, and small organic-chemical molecules with hormone activity, such as thyroid hormone or steroid hormones.

A "neurotransmitter" as used herein is a molecule binding specifically to a neurotransmitter receptor. Examples of well known neurotransmitters are Acetylcholine, Catecholamines, Serotonin, Histamine and certain peptides, among others. Examples of neurotransmitter receptors include opioid-, α2-adrenergic-, dopaminergic-, muscarinic-, cholinergic-, serotonergic- and GABA- receptors.

A "synthetic molecule capable of binding to a surface receptor" as used herein is any small organic chemical molecule found to or designed to bind to the extracellular region of a cellular surface receptor. The binding properties of these small organic chemical molecules for cellular surface receptors are as discussed above for a "component binding specifically to a cell surface molecule".

A "cell type-specific cell surface molecule" as used herein is cell surface molecule which is present at an at least 10 fold, preferably 50 fold, more preferably 200 fold higher density on the cell surface of cells of one specific cell type when compared with cells from at least one other cell type. Preferably, the "cell type-specific cell surface protein" is present at high density in only five, four, three, two or in particular only one specific cell type. Examples for such molecules can be lipids, such as phosphatidylserine, which is only present in the outer leaflets in the case of cell membranes of apoptotic cells, cell type-specific oligosaccharides, for example the sugar moiety of ganglioside GD3 only present on the glia cell lineage, or polypeptides, such as cell type specific receptors such as neurexin III alpha only present on the surface of brain cells, CD4 or CD8, surface molecules only present on subsets of T cells, B220, present on the surface of B cells, CD11c, specifically expressed on dendritic cells, or OSCAR, expressed on the surface of osteoclasts.

A "disorder-specific cell surface protein" as used herein is cell surface molecule which is present at an at least 10 fold, preferably 50 fold, more preferably 200 fold higher density on the cell surface of cells of a cell type affected by a disease when compared with cells from the same cell type in an undiseased state. Examples are virus infected cells of one cell type as compared to uninfected cells of the same cell type, or cancer cells of a particular tissue origin as compared with the corresponding cells of a healthy subject. Examples for such molecules can be lipids, such as phosphatidylserine, which is only present in the outer leaflets in the case of cell membranes of apoptotic cells, which can sometimes be indicative of virus infection, disease-specific oligosaccharides or disease-specific polypeptides, such as cell surface receptors upregulated in certain cancer types, so-called tumor markers (e.g. MCAM, neuropilin, trkA, ErbB-2/HER-2, EGFR, αvβ3 integrin,

CD33, to name but a few). The following reviews deal with cancer therapy using antibodies against tumor cell-specific cell surface markers. The targets of these antibodies would be suitable for the purposes of the invention. Milenic DE. (2002) *Curr. Pharm. Des.* **8,** 1749-1764; Withoff S, Helfrich W, de Leij LF, Molema G. (2001) *Curr. Opin. Mol. Ther.* **3,** 53-62; Penichet ML, Morrison SL. (2001) *J*. *Immunol. Methods.* **248,** 91-101; Weiner LM. (1999) *Semin. Oncol.* **26** (5 Suppl 14), 43-51; Weiner LM. (1999) *Semin. Oncol.* **26** (4 Suppl 12), 41-50.

### DETAILED DESCRIPTION OF THE PREFERRED EMBODIMENTS

The present invention is based on the discovery that adenoviruses of the subgroup B, such as Ad3, use CD46 as their cellular receptor. As detailed in the exemplification section, this finding is the basis for a novel strategy for the modulation of adenovirus subtype B cell binding specificity. The modified polypeptide of the invention now allows the selective infection of desired cell types with recombinant, genetically engineered adenoviruses of subtype B. Such an adenovirus can now be bound, preferably pre-bound, by the adenovirus-binding portion of the modified polypeptides of the invention, the polypeptide derived from the extracellular domain of CD46, and thus be prevented to interact with the CD46 present on the natural target cells of adenovirus subtype B. These polypeptide-bound adenoviruses can then be targeted to a particular cell type of interest by the second functional part of the modified polypeptide of the invention, the cell surface molecule binding component, which can, for example, be a second polypeptide fused to the first by genetic linkage.

Thus, the present invention relates to a modified polypeptide comprising (a) a polypeptide derived from the extracellular domain of CD46 and (b) a component capable of binding to a cell surface molecule. The component (b) is not a polypeptide derived from CD55/DAF. As already mentioned above, the modified polypeptides of the invention display at least two different functions. One is that they are able to bind to an adenovirus of the subgroup B (e.g. Ad3) via the extracellular region of CD46, which provides a specific binding site for those adenoviruses. The second function is to provide an interaction with a selected cell surface molecule, e.g. a cell surface receptor protein. The second function is mediated by component (b), which functionally assures that the modified polypeptide binds specifically to a cell surface molecule of interest. With regard to component (a) of the modified polypeptide, such extracellular domains of CD46, which do not comprise the wild type STP-A region of CD46, are particulary preferred. Preferably, the polypeptide (a) comprises all 4 SCR-regions of CD46, but preferably no other parts of CD 46. Another preferred polypeptide (a) comprises in addition to the four SCR-regions of CD46 also the regions STP-B and STP-C of CD46, but preferably no other parts of CD46.

The polypeptide (a) of the modified polypeptide of the invention can be encoded by a nucleic acid sequence comprising a nucleic acid sequence as defined in SEQ ID NO:12, 14 or 16, it can be encoded by a nucleic acid sequence comprising a nucleic acid sequence that hybridises to the above identified nucleic acid sequences under stringent conditions, it can be encoded by a nucleic acid sequence comprising a nucleic acid sequence which is degenerate as a result of the genetic code to the nucleic acid sequence as defined above, as long as such a nucleic acid sequence still encodes a polypeptide with essentially the same binding activity as the extracellular domain of CD46 for adenovirus of subgroup B, e.g. Ad3, and it can be encoded by a nucleic acid sequence having a sequence identity of at least 70 % with the nucleic acid sequence as defined in SEQ ID NO: 12, 14 or 16, or a fragment thereof, particularly wherein this fragment comprises at least 30, 50, 60, 70, 80, 90 or 100 bases of the nucleic acid sequences as defined in the SEQ ID NO: 12, 14 or 16, as long as the overall nucleic acid sequence encodes a polypeptide having essentially the same binding activity as the extracellular domain of CD46 for an adenovirus of the subgroup B, e.g. Ad3. The binding activity of such a polypeptide (a) for an adenovirus can be determined, e.g. in a FACS-assay like in example 2, wherein a construct, in which the polypeptide (a) substitutes for the extracellular domain of CD46 in the context of the full-length CD46 polypeptide, is transfected into BHK cells and then the binding of adenovirus to the transfected cells is monitored. When binding is assayed by a quantitative assay like a BIACORE binding assay, then a polypeptide is defined to have essentially the same binding activity as the extracellular domain of CD46 for adenovirus of subgroup B, e.g. Ad3, if its dissociation constant for adenovirus is less than 10 fold higher than the dissociation constant of the polypeptide defined in SED ID NO.: 13, when tested in a suitable buffer, like, e.g. the buffer of Example 2.

In particular, the polypeptide (a) can be as defined in the amino acid sequence according to SEQ ID NO: 13, 15 or 17.

Component (b) of the modified polypeptide of the invention can be any molecule which, also in the context of the modified polypeptide of the invention, confers selective binding to a surface molecule. In particular, it can be selected from the group consisting of small organic-chemical molecules, a peptide and a polypeptide, but also nucleic acid aptamers selected for binding to a cell surface molecule can function as a component (b). If component (b) is a peptide, it is preferred that said peptide is derived from a known ligand of a cell surface molecule, e.g. a small portion of a protein with known binding specificity to a cell surface receptor, or it can preferably be derived from a selection scheme, e.g. derived from a phage display screen which selected for cell surface receptor binding peptides, or it can preferably be a peptide designed *in silicio* in such a way that it binds a cell surface receptor. In one preferred embodiment component (b) is a second polypeptide but not a polypeptide derived from a polypeptide of the complement pathway. CD55, e.g., is such a component of the complement pathway. Other proteins, from which component (b) is preferably not derived, are C1NIH, C4BP, CR1, THF, H, I, P and CD59.

In another preferred embodiment, the small organic-chemical molecule is selected from the group consisting of a non-proteinatious hormone, a neurotransmitter and a synthetic molecule capable of binding to a cell surface receptor. E.g. a modified polypeptide comprising the extracellular domain of CD46 linked with serotonin would target recombinant adenoviruses to cells displaying the serotonin receptor. This is to exemplify that any component (b), which functions in the context of the modified polypeptide of the invention to target an adenovirus of subtype B of the surface of a cell of interest, such as e.g. a nerve cell, can be useful in the context of the bifunctional modified polypeptide of the invention. The extracellular domain of CD46 binds adenoviruses of subtype B with high affinity. It is therefore desirable that also the component (b) of the modified polypeptide of the invention is able to bind a cell surface molecule, such as a cell surface receptor, specifically and with high affinity, such as an affinity constant of lower than 1 µM, preferably between 0,01 nM and 500 nM, more preferably from 0,1 nM to 200 nM.

In a preferred embodiment the component (b) of the modified polypeptide of the invention can bind, and preferably binds specifically, a molecule selected from the group consisting of a cell type specific cell surface molecule, a disorder-specific cell surface specific molecule, a cell surface receptor, a cell adhesion molecule and a sugar moiety located on one of the aforementioned molecules. Particularly, such binding partners of component (b) can be molecules selected from the group consisting of a leukocyte antigen, a receptor tyrosine kinase, like the EGF receptor, a receptor of the TNF receptor family, a cytokine receptor, like the EPO receptor, the IL-2 receptor and IFN receptors, a G-protein coupled receptor, like the Bradikinin receptor and the beta-endorphin receptor, a receptor tyrosine phosphatase, like the receptor tyrosin phosphatase zeta, a chemokine receptor, like CCR7, a scavenger receptor, like CD36, a Fc receptor, like CD16 and CD32, a tetraspannin, like CD37, a member of the Ig-superfamily, like CD1 and CD33, an ion channel, like the acetylcholine receptor, and a lectine.

In a preferred embodiment, component (b) is an antibody or an antibody fragment. It is clear that an antibody against a cell surface molecule can very well be used as a component (b) in the context of a modified polypeptide of the invention. Numerous antibodies selective against cell surface markers have been described. Of particular usefulness are those antibodies or antibody fragments which can bind selectively to one of the before mentioned cell surface molecules, e.g. cell surface receptors, cell adhesion molecules, disorder specific cell surface molecules or cell type specific cell surface molecules, particularly such antibodies or antibody fragments which have proven useful for the detection of so-called tumor markers (e.g. MCAM, neuropilin, trkA, ErbB-2/HER-2, EGFR, αvβ3 integrin, CD33, to name but a few). The following reviews deal with cancer therapy using antibodies against tumor cell-specific cell surface markers. The targets of these antibodies would be suitable for purposes of the invention. Milenic DE. (2002) *Curr. Pharm. Des.* **8**, 1749-1764; Withoff S, Helfrich W, de Leij LF, Molema G. (2001) *Curr. Opin. Mol. Ther.* **3**, 53-62; Penichet ML, Morrison SL. (2001) *J. Immunol. Methods.* **248**, 91-101; Weiner LM. (1999) *Semin. Oncol.* **26** (5 Suppl 14), 43-51; Weiner LM. (1999) *Semin. Oncol.* **26** (4 Suppl 12), 41-50.. Several antibody fragments, which retain specific binding activity, have been described in the art and those modified polypeptides, wherein component (b) is one of an scFab, Fab, F(ab')₂, diabody, or an scFv are preferred modified polypeptides of the invention. Most preferred are scFvs, particularly such scFvs which selectively bind tumor markers.

In a particularly preferred embodiment component (b) is an antibody fragment, and particularly an scFv, which binds specifically to ErbB-2, like the scFv-FRP5 of example 7, the EGF receptor, like the scFv-225 or the scFv-R1 of example 7, the calcium channel CD20, the ganglioside GD3, the VEGF receptor-2 (KDR), the tumor associated glycoprotein TAG-72, the epithelial cell adhesion molecule Ep-CAM (17-1A antigen), the cell surface glycoprotein CEA or the sialic acid binding I-type lectin CD33. These cell surface molecules are selectively upregulated in certain tumor cells: ErbB-2 is overexpressed in 30% of all breast and ovarian cancers, EGFR is overexpressed in many tumors of epithelial origin, like lung, breast, head and neck or bladder cancer, CD20 is selectively expressed in B cell lymphoma, GD3 is a surface antigen selective for certain tumor cells, like small cell lung carcinoma, KDR is found almost exclusively on growing endothelial cells, TAG-72 is a selective surface marker of most adenocarcinomas, Ep-CAM is selectively overexpressed in colon and colorectal cancer, CEA is a relevant tumor marker in colorectal cancer and breast cancer and CD33 is found in cancerogenous cells in AML, the most commonly diagnosed type of leukaemia in adults. Thus, modified polypeptides of the invention in which component (b) is an antibody fragment, particularly an scFv, against these aforementioned molecules can be used together with recombinant adenoviruses for the treatment of the diseases associated with the overexpression of said aforementioned cell surface markers.

Other molecules, which can fulfil the functional requirement for a component (b) useful in the context of a modified polypeptide of the invention are such polypeptides, which are known to be ligands of cell surface molecules, particularly those selected from the group consisting of a ligand of cell type specific cell surface molecules, a ligand of a disorder specific cell surface molecule, a ligand of a cell surface receptor, a ligand of a cell adhesion molecule and a ligand of a sugar moiety located on one of the before mentioned molecules, in particular such ligands selected from the group consisting of a ligand of a leukocyte antigen, a ligand of a receptor tyrosine kinase, a ligand of a receptor of the TNF-receptor family, a ligand of a cytokine receptor, a ligand of a G-protein coupled receptor, a ligand of a receptor tyrosine phosphatase, a ligand of a chemokine receptor, a ligand of a scavenger receptor, a ligand of a Fc-receptor, a ligand of a tetraspannin, a ligand of a member of the Ig-superfamily and a lectine.

Particularly preferred are such ligands which have been modified, so that they still bind to their particular binding partner, e.g. a cell surface receptor, but no longer elicit the typical cellular response to such binding, e.g. a VEGF-mutant, which still binds to the VEGF-receptor, but does not induce VEGF-receptor signalling. For example, in cases where signalling is elicited by receptor dimerization after binding to a bivalent ligand, monovalent ligands may be an example of such ligands, which bind, but do not induce signalling.

Examples of ligands, which may be used as a component (b) of the invention, are polypeptides or peptides derived from interferons, insulin, growth-factors, like NGF, EGF, PDGF, members of the neurotrophin family, TGF-α, FAS, CD40, IGF-1, IGF-2, TGF-β, BMPs, IL-2, BDNF, NT-3, NT-4, erythropoietin, inter-leukin-3, CSFs, bradikinin, beta-endorphin, adiponectin and the like. Particularly preferred are such mutants of these proteins, which still bind to their receptor, but elicit a significantly, like 10-fold, 50-fold or 200-fold lower cellular response as compared to their wild type counterpart, or even no response at all.

The two components of the modified polypeptide of the invention, the polypeptide (a) and the component (b) can be linked together indirectly, e.g. via two molecules, which strongly interact, like the biotin/streptavidin system, or directly, i.e. by a covalent linkage between (a) and (b). The covalent linkage between the two components (a) and (b) can be effected by chemical cross linking, thus linking two components together, which were initially produced independently, or also by genetic fusion, that is to say that, in case where component (b) is a polypeptide, the nucleic acids encoding the polypeptides (a) and (b) are combined by genetic engineering techniques in such a way that the expression of this combined nucleic acid leads to a polypeptide comprising both, a polypeptide (a) and a polypeptide (b) on the same polypeptide chain. Polypeptide (b) can be N-terminal of polypeptide (a), however, it is preferred that polypeptide (a) is N-terminal of polypeptide (b). The two polypeptides may directly follow one another or they may have a linker sequence inserted between them. In a preferred embodiment, the polypeptide (a) is not preceded by any further N-terminal sequence.

When the polypeptide (a) and the component (b) are chemically crosslinked, they can be crosslinked by a spacer, wherein the spacer is selected from the group consisting of functional cross-linkers, flexible amino acid linkers, like the hinge region of immunoglobulins, glycine serine linkers and glycine linkers and homobifunctional crosslinkers. As mentioned above, also stable ligand-receptor pairs, like e.g. the biotin/streptavidin system, can be used to link polypeptide (a) and component (b). For the present invention, preferred linkers are heterobifunctional cross-linkers. Several hetero-bifunctional cross-linkers are known to the art. These include the preferred cross-linkers SMPH (Pierce), Sulfo-MBS, Sulfo-EMCS, Sulfo-GMBS, Sulfo-SIAB, Sulfo-SMPB, Sulfo-SMCC, SVSB, SIA and other cross-linkers available for example from the Pierce Chemical Company (Rockford, IL, USA). A preferred cross-linker has one functional group reactive towards amino groups and one functional group reactive towards cysteine residues. The above mentioned preferred cross-linkers all lead to formation of a thioether linkage. Another class of cross-linkers suitable in the practice of the invention is characterized by the introduction of a disulfide linkage between the polypeptide of (a) and the component (b) of the chimeric polypeptide of the invention. Preferred cross-linkers belonging to this class include for example SPDP and Sulfo-LC-SPDP (Pierce).

Flexible amino acid linkers are further preferred embodiments. Examples of the amino acid linker are selected from the group consisting of: (a) CGG; (b) N-terminal gamma 1-linker; (c) N-terminal gamma 3-linker; (d) Ig hinge regions; (e) N-terminal glycine linkers; (f) (G)ₖC(G)ₙ with n=0-12 and k=0-5; (g) N-terminal glycine-serine linkers; (h) (G)ₖC(G)ₘ(S)ₗ(GGGGS)ₙ with n=0-3, k=0-5, m=0-10, l=0-2; (i) GGC; (k) GGC-NH2; (l) C-terminal gamma 1-linker; (m) C-terminal gamma 3-linker; (n) C-terminal glycine linkers; (o) (G)ₙC(G)ₖ with n=0-12 and k=0-5; (p) C-terminal glycine-serine linkers; (q) (G)ₘ(S)ₗ(GGGGS)ₙ(G)ₒC(G)ₖ with n=0-3, k=0-5, m=0-10, l=0-2, and o=0-8.

Further examples of amino acid linkers are the hinge region of Immunoglobulins, glycine serine linkers (GGGGS)ₙ, and glycine linkers (G)ₙ all further containing a cysteine residue and optionally further glycine residues. Typically preferred examples of said amino acid linkers are N-terminal gamma1: CGDKTHTSPP; C-terminal gamma 1: DKTHTSPPCG; N-terminal gamma 3: CGGPKPSTPPGSSGGAP; C-terminal gamma 3: PKPSTPPGSSGGAPGGCG; N-terminal glycine linker: GCGGGG and C-terminal glycine linker: GGGGCG.

Other amino acid linkers particularly suitable in the practice of the invention, are CGKKGG, or CGDEGG for N-terminal linkers, or GGKKGC and GGEDGC, for the C-terminal linkers. For the C-terminal linkers, the terminal cysteine is optionally C-terminally amidated.

In preferred embodiments of the present invention, GGCG, GGC or GGC-NH2 ("NH2" stands for amidation) linkers at the C-terminus of polypeptide (a) or CGG at its N-terminus are preferred as amino acid linkers. In general, glycine residues will be inserted between bulky amino acids and the cysteine, to avoid potential steric hindrance of the bulkier amino acid in the coupling reaction.

Further preferred cross-linkers are the carbodiimide EDC, and NHS, as well as homo-bifunctional cross-linker such as glutaraldehyde, DSG, BM[PEO]₄, BS³, (Pierce Chemical Company, Rockford, IL, USA) or other known homo-bifunctional cross-linkers preferably reactive towards amine groups or carboxyl groups of the polypeptide.

Further preferred methods of crosslinking include methods where the polypeptide (a) or component (b) of the chimeric polypeptide of the invention is biotinylated, and the other corresponding component is expressed as a streptavidin-fusion protein, or methods wherein both components (a) and (b) are biotinylated. In this case, the polypeptide (a) may be first bound to streptavidin or avidin by adjusting the ratio of the polypeptide (a) to streptavidin such that free binding sites are still available for binding of the component (b) of the chimeric polypeptide, which is added in the next step. Alternatively, all components may be mixed in a "one pot" reaction. Other ligand-receptor pairs, where a soluble form of the receptor and of the ligand is available, and are capable of being cross-linked to the polypeptide (a) or component (b) of the chimeric polypeptide, may be used as binding agents for binding the polypeptide (a) to the component (b) of the chimeric polypeptide. Alternatively, either the ligand or the receptor may be fused to the polypeptide (a), and so mediate binding to the component (b) chemically bound or fused either to the receptor, or the ligand respectively. Fusion may also be effected by insertion or substitution.

In a preferred embodiment, the modified polypeptide is a multimer , particularly a dimer, trimer or tetramer when analyzed by gel-filtration chromatography. This can be due to the nature of component (b). Alternatively, polypeptide or peptide sequences which lead to the formation of dimers, trimers or tetramers can be comprised by the modified polypeptide of the invention. Particularly preferred are trimers.

In a particularly preferred embodiment a modified polypeptide of the invention is defined as in the amino acid sequence according to SEQ ID NO: 19 or 21.

Another embodiment of the invention is a nucleic acid comprising a nucleic acid sequence encoding a modified polypeptide of the invention, e.g. a nucleic acid encoding a fusion polypeptide comprising a polypeptide sequence derived from the extracellular domain of CD46 and also comprising a second polypeptide sequence which can function as a component (b) and is capable of binding to a cell surface molecule, e.g. cell type specific cell surface receptors and disease-specific cell surface receptors.

In a preferred embodiment, the nucleic acid comprises (i) a nucleic acid sequence as defined in the SEQ ID NO: 12, 14 or 16, (ii) a nucleic acid sequence which hybridizes to nucleic acid sequences as defined in (i) under stringent conditions, (iii) a nucleic acid sequence which is degenerate as a result of the genetic encode to the nucleic acid as defined in (i) and (ii) and which encodes a polypeptide having essentially the same binding activity as the extracellular of CD46, or (iv) a nucleic acid sequence having a sequence identity of at least 70 % with the nucleic acid sequence as defined in (i) or a fragment thereof, preferably wherein the fragment is at least 30, 40, 50, 60, 70, 80, 90 or 100 bases long, and which encodes a polypeptide having essentially the same binding activity as the extracellular domain of CD46. The term "stringent" as used herein means washing conditions of 1 x SSC, 0.1 % SCS at a temperature of 65°C, preferably the SCC concentration is 0,3 x SSC. The first two washings are carried out twice for 15-30 min. Hybridization can be performed e.g. overnight in 0,5 M phosphate buffer, pH 7,5/7 % SDS at 65°C. Hybridization methods are as disclosed in the standard text book on molecular cloning: Molecular Cloning: A Laboratory Manual, 3^{rd} ed. EDS Sambrook et al., CSHL press, 2001.

A further embodiment of the invention is of a recombinant expression vector comprising a nucleic acid of the invention which is operably linked to at least one regulator sequence allowing expression of the modified protein of the invention. For example, a nucleic acid sequence encoding a modified polypeptide of the invention can be isolated and cloned into an expression vector and the vector can then be transformed into a suitable host cell for expression of a modified polypeptide of the invention. Such a vector can be a plasmid, a phagemid or a cosmid. For example, a nucleic acid molecule of the invention can be cloned in a suitable fashion into prokaryotic or eukaryotic expression vectors (Sambrook et al., supra).

Such expression vectors comprise at least one promoter and can also comprise a signal for translation initiation and - in the case of prokaryotic expression vectors - a signal for translation termination, while in the case of eukaryotic expression vectors preferably expression signals for transcriptional termination and for polyadenylation are comprised. Examples for prokaryotic expression vectors are, for expression in Escherichia coli e.g. expression vectors based on promoters recognized by T7 RNA polymerase as described in US 4,952,496, for eukaryotic expression vectors for expression in Saccharomyces cerevisiae, e.g. the vectors G426/Met25 or P526/Gal1 (Mumberg et al., (1994) Nucl. Acids Res., 22, 5767-5768), for the expression in insect cells, e.g. Baculovirus vectors, as e.g described in EP-B1-0127839 or EP-B1-0549721 or by Ciccarone et al. ("Generation of recombinant Baculovirus DNA in E.coli using baculovirus shuttle vector" (1997) Volume 13, U. Reischt, ed. (Totowa, NJ: Humana Press Inc.) and for the expression in mammalian cells, e.g. the vectors Rc/CMW and Rc/ASW and SW40-Vectors, which are commonly known and commercially available, or the EBNA-system described in Example 4, the Sindbis replicon-based pCytTS (Boorsma et al. (2002) Biotechnol. Bioeng. 79(6): 602-609), the Sindbis virus expression system (Schlesinger (1993) Trends Biotechnol. 11(1):18-22) or an Adenovirus expression system (He et al. (1998) Proc. Natl. Acad. Sci. USA 95:2509-2514). The molecular biological methods for the production of these expression vectors as well as the methods for transfecting host cells and culturing such transfected host cells as well as the conditions for producing and obtaining the polypeptides of the invention from said transformed host cells are well known to the skilled person.

The present invention further relates to a host cell comprising a nucleic acid of the invention and/or a recombinant expression vector of the invention, particularly wherein the host cell is a microorganism like yeast or other fungi, like Escherichia coli, Bacillus subtilis or other bacteria. The host cell can also be a cell of higher eukaryotic origin, like an insect cell, preferably a virus infected insect cell, more preferably a Baculovirus infected insect cell, or like a mammalian cell like HeLa, COS, NDCK, 393, EBNA-1, NS0 or a hybridoma cell, preferably a cell selected from the group consisting of a monocellular phagocyte lineage cell, 293 cells, BHK cells and Sf9 cells.

A further embodiment of the invention is a pharmaceutical composition comprising a modified polypeptide of the invention and a pharmaceutically acceptable carrier. Pharmaceutically acceptable carriers are all inert, non-toxic, liquid or solid fillers or diluents, as long as they do not react with the polypeptide of the invention in an inappropriate negative manner. Liquid pharmaceutical carriers are, for example, sterile water, saline, sugar solutions, ethanol and/or certain oils. Carriers for the production of capsules and tablets may include binders and fillers. Preferably also a recombinant adenovirus of subtype B, e.g. Adenovirus 3, is included in said pharmaceutical composition.

The invention further provides a method of producing a modified polypeptide of the invention, which method comprises the culturing of a host cell of the invention under conditions suitable for expression of the modified polypeptide in the host cell and isolating the modified polypeptide from the host cell. In an additional step the isolated modified polypeptide is further formulated as a pharmaceutical composition.

The invention further provides a method for treating a patient in need of such treatment or preventing a patient from acquiring a disease, wherein the patient is administered a therapeutically effective amount of a modified polypeptide of the invention together with a recombinant adenvirus of the subtype B. Preferably, the polypeptide of the invention is in molar excess over the recombinant adenovirus, even more preferably in a more than 12-fold molar excess over the recombinant adenovirus, and most preferably in such a molar excess that all CD46-binding sites of the recombinant adenovirus are saturated. This will assure that the adenovirus will not infect normal target cells, but will infect those cells to which it is targeted via the binding specificity of component (b) of the polypeptide of the invention. The recombinant adenovirus may be used from 10⁵ to 10¹² plaque forming units. Administration may be systemically or locally, for example targeting specifically one particular organ, like, e.g., the lung.

The invention further provides modified polypeptides of the invention for use in medicine. In one embodiment a polypeptide comprising only a functional polypeptide (a), but no functional component (b) is provided for the use in medicine, like the treatment of disorders or diseases caused by adenovirus subtype B infection, like respiratory and gastrointestinal infections. Such a polypeptide derived from the extracellular domain of CD46 only and with no functional component (b), will compete for binding of adenovirus to normally susceptible cells. Also the above mentioned pharmaceutical composition comprising such a polypeptide is provided by the invention. Adenoviral subtype B-infection plays a role in the following disorders or diseases: respiratory disorders or diseases, like certain types of pneumonia; pharyngokonjunktival fever; epidermal keratokonjunktivitis; gastroenteritis with respiratory-tract infection, which can be accompanied by appendizitis in infants and toddlers; rare forms of an encephalitis and certain types of exanthemes. Thus, the use of a polypeptide derived from the extracellular domain of CD46, but with no functional component (b), which would target it to a cell-surface, for the preparation of a medicament for the treatment of disorders and diseases caused by adenovirus subtype B infection, like the disorders and the diseases mentioned above, is an embodiment of the invention.

For the treatment of other diseases, however, the modified polypeptides of the invention comprising a component (a) and (b) as defined above in combination with a recombinant adenovirus can be used. Therefore, in another embodiment, the modified polypeptides of the invention are used in combination with a recombinant adenovirus of the subtype B, e.g. adenovirus 3 or 7. More preferably, the polypeptide of the invention is in molar excess over the recombinant adenovirus, even more preferably in a more than 12-fold molar excess over the recombinant adenovirus, and most preferably in such a molar excess that all CD46-binding sites of the recombinant adenovirus are saturated. This will assure that the adenovirus will not infect normal target cells, but will infect those cells to which it is targeted via the binding specificity of component (b) of the polypeptide of the invention. An example for such a re-targeting of adenovirus is provided in Example 7, where Ad3 is redirected to ErbB2-expressing cancer cells by means of a CD46-FRP5 fusion protein. The use of recombinant adenovirus for gene therapy is well known in the art and described, e.g., in a series of articles, like Loser et al., (2002) Curr Gene Ther. 2(2):161-71; Mitani and Kubo, (2002) Curr Gene Ther. 2(2):135-44; VandenDriessche et al., (2001) Curr Gene Ther. 1(3):301-15; Bronte V. (2001) Curr Gene Ther. 1(1):53-100; Ochiya et al., (2001) Curr Gene Ther. 1(1):31-52; Wu et al., (2001) Curr Gene Ther. 1(1):101-22 and Breyer et al., (2001) Curr Gene Ther. 1(2):149-62.

In a further embodiment the invention provides a pharmaceutical composition comprising a modified polypeptide of the invention and a pharmaceutically acceptable carrier, preferably further comprising an adenovirus of the subtype B or another virus, which other virus expresses a molecule capable of binding a CD46 extracellular domain, which adenovirus or virus has been genetically engineered. In a preferred embodiment the concentrations of the polypeptide of the invention is in molar excess over the recombinant adenovirus or said other virus, even more preferably in a more than 12-fold molar excess over the recombinant adenovirus or said other virus, and most preferably in such a molar excess that all CD46-binding sites of the recombinant adenovirus or said other virus are saturated. This will assure that the adenovirus or said other virus will not infect normal target cells, but will infect those cells to which it is targeted via the binding specificity of component (b) of the polypeptide of the invention. In a preferred embodiment said adenovirus or virus has been genetically engineered by introducing a therapeutically active gene construct, which is preferably operably linked to at least one regulatory sequence for expression of the therapeutically active gene.

WO0032754 explains how this operable linkage can be performed such that the therapeutically active gene construct is expressed in the cells infected by the recombinant adenovirus.

WO0029024 explains how recombinant adenoviruses can be formulated for gene therapy.

In a preferred embodiment said therapeutically active gene is a tumor suppressor gene. Tumor suppressor genes, potentially suitable for gene therapy, are reviewed in Macleod K. (2000) *Curr. Opin. Genet. Dev.* **10**, 81-93; Munger K. (2002) *Cancer Invest.* **20**, 71-81; Teh BT, Larsson C, Nordenskjold M. (1999) *Anticancer Res.* **19**, 4715-4728. Particularly preferred are the tumor suppressor genes selected from the group consisting of p53 (such as ONYX-015 from Onyx Pharmaceuticals), Retinoblastoma, NF2, BRCA1, BRCA2, MSH2, MSH6, MLH1, CDKN2, Apafl, DPC4, PKD1, HPC1 and VHL.

In a further embodiment this pharmaceutical composition is used for the manufacture of a pharmaceutical for the treatment of a disorder or a disease which can be treated by supplying a therapeutically active gene construct. Such diseases can either be characterized in that they are caused by a mutation in the genome of an affected individual, which mutation leads to reduced or even abolished expression of a certain functional gene product, e.g. decreased or abolished expression of certain tumor suppressor genes in certain types of cancer. Preferably, the disease is selected from the group consisting of SCID, cystic fibrosis, arthritis, multiple sclerosis and cancer, in particular cancer deficient in any one tumor suppressor gene, like lung cancers deficient in proper p53 expression. These disorders or diseases can be treated by directing a recombinant adenovirus supplying the proper gene product to the cell type in need of that gene product, for example by directing a p53-expressing recombinant adenovirus to the p53-deficient lung cancer cells in the above example, BRCA1-expressing recombinant adenovirus to a BRCA1-deficient breast cancer cell. Other such examples of cancers, where deficiency of a tumor suppressor gene has been linked to the disease, and which can be treated by the combined pharmaceutical composition of the invention, are (tumor suppressor in brackets) Retinoblastoma (Rb); Wilms tumor (Wtl); Sarcomas, breast tumors and certain brain tumors (p53); Colon cancer (APC); breast and ovarian tumors (BRCA1, BRCA2); breast, colon, skin and lunc carcinoma (E-Cadherin); Prostate (NKX3.1) and other examples reviewed in Macleod K. (2000) *Curr. Opin. Genet. Dev.* **10**, 81-93).

### DESCRIPTION OF THE FIGURES

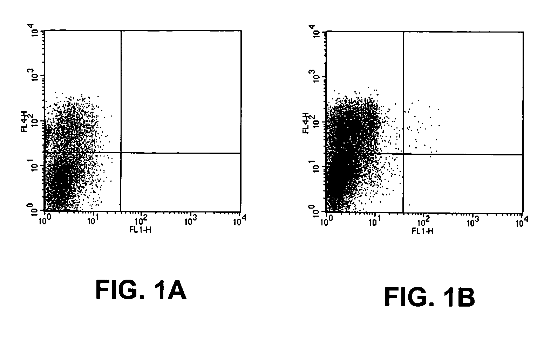

Figure 1: Single cell sorting. BHK cells were infected with the normalized K562 viral library (Panel B) or with sindbis virus as a control (Panel A). Infected cells were stained with PI to exclude dead cells, and with anti-Sindbis and Alexa 488nm-labeled Ad3. PI-negative, Sindbis (FL4; Y axis)- and Ad3 (FL1; X axis)-positive cells in the indicated gate were single cell sorted into 24-well plates containing BHK feeder cells.

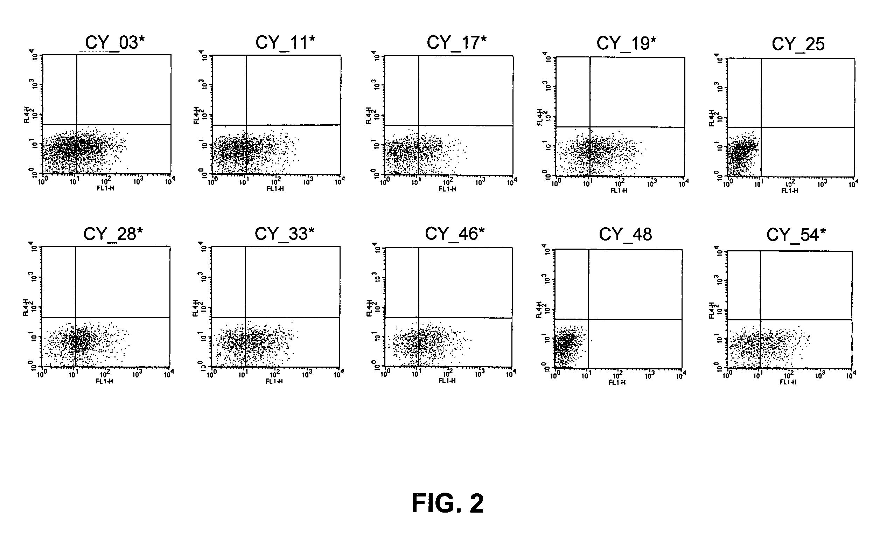

Figure 2: Rescreen of Ad3-specific single cell sorting. Single cells were sorted into 24-well plates. Supernatants of wells showing typical signs of viral infection were collected, the cells were stained with the Alexa 488nm-labeled Ad3 and analyzed by FACS. X axis, FL1 (Ad3 signal); Y axis, FL4. Clone numbers are given on top of each panel. Positive clones are marked with an asterisk.

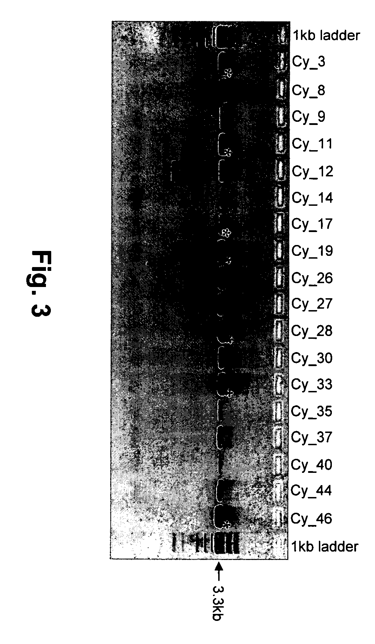

Figure 3: Rescue of the Ad3 receptor cDNA. Viral supernatants from the 64 clones that were positive in the rescreen were subjected to RT-PCR followed by agarose gel electrophoresis. A representative analysis of 18 clones is shown. The predominant bands migrated at approx. 3.3kb. The bands marked with an asterisk were excised from the gel, sequenced, and turned out to encode CD46 splice variants (see Table I).

### DESCRIPTION OF THE TABLE

Table 1 is an Overview of CD46 clones obtained from the Ad3 screening after the steps described in Example 3. The following legend is provided:
* Exon present but not translated (i.e. not present in ORF)
**15bp missing leading to 5aa deletion
SCR = short complement like repeat, a.k.a. CCP (complement control protein) domain
STP = serine/threonine/proline-rich region
TM = transmembrane region
CYT = cytoplasmic domain

### DESCRIPTION OF THE SEQUENCES

SEQ ID NO: 1 gives the DNA sequence of the full length human CD46 open reading frame.

SEQ ID NO: 2 specifies the full length human CD46 polypeptide and describes the domain structure of this protein.

SEQ ID NO: 3 specifies the polypeptide sequence of the STP-A region, which is present in certain splice variants of CD46.

SEQ IDs NO: 4-11 detail the nucleic acid sequences of the selected open reading frames.

SEQ IDs NO: 12, 14 and 16 describe the nucleic acid sequences of several selected clones encoding extracellular domains of CD46.

SEQ IDs NO: 13, 15 and 17 describe the corresponding encoded amino acid sequences.

SEQ ID NO: 18 describes the nucleic acid sequence of a construct encoding a fusion polypeptide between the extracellular domain of CD46 at the N-terminus and a human Fc-gamma3 domain at the C-terminus.

SEQ ID NO: 19 is the polypeptide sequence encoded by SEQ ID NO:18.

SEQ ID NO: 20 describes the nucleic acid sequence of a construct encoding a fusion polypeptide between the extracellular domain of CD46 at the N-terminus and the ErbB-2-binding scFv FRP5 at the C-terminus.

SEQ ID NO: 21 is the polypeptide sequence encoded by SEQ ID NO:20.

### EXAMPLES

### Example 1.

### Construction of a normalized alphaviral cDNA expression library containing the Ad3 receptor

PolyA+ RNA was isolated from the K562 human myelogenous leukaemia cell line with the FastTrack™ mRNA isolation kit (Invitrogen). Single-stranded cDNA was produced from 2 µg polyA+ RNA with PowerScript™ reverse transcriptase (Clontech) using the template switch protocol (Zhu et al., 2001), with the 3'-Sfi oligonucleotide (5'-AAG CAG TGG TAT CAA CGC AGA GTG GCC GAG GCG GCC TTT TTT TTT TTT TTT TTT TTT TTT TTT TTT VN-3') as primer, and the 5'-Sfi oligonucleotide (5'-d[AAG CAG TGG TAT CAA CGC AGA GTG GCC ATT ACG GCC] r[GGG]-3') as switch template.

The first strand cDNA was prepared for use as tester in the normalization procedure as follows. First, the reaction was extracted with phenol-chloroform and ethanol precipitated twice in the presence of 2M ammonium acetate. Then, the resulting pellet was resuspended in 0.5M NaOH and incubated at 55°C for 15 minutes to degrade the RNA. Finally, the cDNA was ethanol precipitated in the presence of 0.3M sodium acetate and resuspended in water.

K562 polyA+ RNA was prepared for use as driver by photobiotinylation. Thus, 10 µg RNA were mixed with 30 µg photobiotin acetate and irradiated for 20 minutes with strong visible light, using a 300W Phillips Reflector Floodlamp. Free photobiotin was then removed by 2-butanol extraction in the presence of 25mM Tris pH 9. After ethanol precipitation, the RNA was resuspended in water, and the photobiotinylation was repeated. Free photobiotin was removed by five 2-butanol extractions in the presence of 25mM Tris pH 9. Finally, the RNA was ethanol precipitated and resuspended in water for use as a driver.

For hybridization, 2 µg single-stranded tester cDNA were mixed with 2 µg biotinylated driver polyA+ RNA and 0.2 µg oligo-dT (20mer), ethanol precipitated, and resuspended in 25 µl formaldehyde hybridization buffer (80% formaldehyde, 25 mM Hepes pH 7.5, 250 mM NaCl, 5 mM EDTA). Hybridization was carried out in an Eppendorf Mastercycler personal. After an initial denaturation step for 5 minutes at 65°C, nucleic acid hybridization was allowed to proceed for 16 hours at 42 °C. After this, the reactions were immediately chilled on ice, and the volume was adjusted to 100µl with 10 mM Hepes pH 7.5, 1 mM EDTA. Unhybridized single-stranded cDNA was purified by streptavidin-mediated removal of RNA/DNA hybrids and excess driver RNA. Thus, 10 µl streptavidin (1mg/ml) was added to the hybridization mixture and incubated for 10 minutes at room temperature, after which the solution was extracted with phenol-chloroform. After an additional round of streptavidin binding and extraction, the remaining single-stranded cDNA was ethanol precipitated and resuspended in 20 µl H₂O.

Double-stranded cDNA was then produced by 14 cycles of polymerase chain reaction (PCR), using the Advantage2 polymerase mix (Clontech) and an anchor primer (5'-AAG CAG TGG TAT CAA CGC AGA GT-3') in a total volume of 500 µl. Double-stranded cDNA was purified with the Qiaquick PCR purification kit (Qiagen), digested with the restriction endonuclease Sfi1 (Roche), and sizefractionated by agarose gel electrophoresis. Three fractions, corresponding to large (>3kb; fraction A), intermediate (1.5-3kb; fraction B), and small cDNAs (0.4-1.5kb; fraction C) were isolated by electroelution and cloned separately into the alphaviral expression vector pDelSfi. Each of the sublibraries consisted of >10⁷ independent transformands. DNA was isolated from pooled colonies using the HiSpeed Plasmid Maxi Kit (Qiagen).

Plasmids were prepared for in vitro transcription as follows. 5 µg of each sublibrary were linearized, half with the restriction endonuclease Not1 (Roche), the other half with Pac1 (New England Biolabs). 5 µg of the helper plasmid pDHEB (Bredenbeek et al., 1993), encoding the Sindbis virus structural proteins, were linearized with the restriction endonuclease EcoR1. All restriction digests were then extracted with phenol-chloroform, ethanol precipitated, and resuspended in RNase-free H₂O at a concentration of 0.5 µg/µl. 1 µg of each linearized sublibrary and of the helper plasmid were subjected to SP6 RNA polymerase-mediated in vitro transcription in a volume of 20 µl, using the mMessage mMachine™ kit (Ambion). Each sublibrary RNA was co-electroporated with an equimolar amount of helper RNA into 10⁷ BHK cells. 18 hours post transfection, cell supernatants were harvested and the viral titers determined. Titers were: fraction A, 4x10⁶; fraction B, 2.8x10⁶; fraction C, 3x10⁶. This Sindbis virus based cDNA expression library was then used in Example 2 in a screen for the cellular receptor of adenovirus, subtype B.

### Example 2.

### Identification of Ad3 receptor expressing cells by fluorescence-activated cell sorting

Subconfluent (80%) baby hamster kidney (BHK) cells, which do not express a receptor for adenovirus subtype B and can, by themselves, not be bound and infected by these adenoviruses, were infected with the normalized K562 viral library (the Sindbis virus based cDNA expression library of Example 1) at a multiplicity of infection (MOI) of 0.1. 10⁷ cells were infected with each sublibrary. After 2 hours, cells were washed once and incubated for another 6 hours in the presence of a neutralizing mouse anti-Sindbis antiserum to avoid superinfection. 8 hours post-infection cells were detached with cell dissociation buffer (PBS-EDTA, Gibco BRL), washed and stained for 30 min with Alexa 488nm-labeled Adenovirus Ad3 (diluted 1:10) and Cy5-labelled anti-mouse Ig (diluted 1:400). Cell pools were then filtered and stained with propidium iodide (PI) to exclude dead cells. Single cell sorting was performed on a FACS Vantage flow cytometer (Becton Dickinson), sorting for Alexa 488nm-positive, Sindibis-positive (by the presence of Cy5) and PI negative cells (Figure 1). In total, 238 cells were sorted into single wells of 24-well plates. The rationale behind this screen is that only those cells, which after Sindbis virus infection express a specific cDNA coding for an adenovirus receptor, can bind to the Alexa-labelled Adenovirus Ad3.

Each sorted single cell, which potentially habors a recombinant Sindbis virus with a cDNA coding for the adenovirus receptor, was incubated in a well of a 24-well plate containing 50% confluent BHK feeder cells. Upon virus spread (2-3 days post-sorting), the infected cells were tested by FACS analysis for binding of Alexa-labelled Adenovirus. At day 2, 63 wells showed typical signs of adenoviral infection and 42 bound the Alexa 488 nm-labeled Adenovirus. One day later, at day 3 post sorting procedure, another 40 wells showed clear adenoviral infection and 22 of them bound the Adenovirus (Figure 2). Some of these positive samples were further processed for gene rescue.

### Example 3.

### Rescue of cDNA encoding the Ad3 receptor, CD46

To obtain the cDNA encoding a putative Ad3 receptor, a RT-PCR was performed using 8 supernatants, each containing monoclonal recombinant sindbis virus.

For the viral RNA isolation 140 µl of viral supernatant and OIAmp Viral RNA Kit (Qiagen, Cat No.: 52409) was used. The procedure was performed according to manufacturer's protocol and the RNA was dissolved in 30 µl DEPC-H₂O

For the cDNA synthesis 9 µl of the viral RNA were used in a reaction. The 1^{st} strand cDNA was synthesized in a reaction containing 50 mM Tris-HCl, pH 8.3, 75 mM KCl, 3 mM MgCl2, 10 mM dithiothreitol, 500 µM dATP, dCTP, dGTP, dTTP, 2 pmol LPP2 primer (5'- ACA AAT TGG ACT AAT CGA TGG C-3'), 40 Units RNaseOUT (Invitrogen Life Technologies, Cat. No. 10777-019), and 200 Units SUPERSCRIPT™ II RNase H- reverse transcriptase (Invitrogen Life Technologies, Cat. No. 18064-022) in a total volume of 20 µl at 42°C for 1 hour. Following the reverse transcription the reaction was terminated by incubation at 85°C for 5 minutes. To remove the complementary RNA prior to PCR the cDNA was treated with 2 Units of RNase H at 37°C for 30 minutes.

The PCR was performed using cDNA as template, High Fidelity PCR System composed of unique enzyme mix containing thermostable Taq DNA polymerase and a proofreading polymerase (Roche, Cat. No. 1 732 650) and the primers GW-LPP1 (5'- GGG GAC AAG TTT GTA CAA AAA AGC AGG CTA TAC GAC TCA CTA TAG GGA GAC-3') and GW-LPP2 (5'- GGG GAC CAC TTT GTA CAA GAA AGC TGG GTA CAA ATT GGA CTA ATC GAT GGC-3'). The PCR reaction was performed on a Hybaid programmable thermal cycler with one cycle of 2 min at 95°C; 5 cycles of 30sec at 94°C, 45 sec at 57°C, 70 sec at 68°C; 25 cycles of 30 sec at 94°C, 70 sec at 68°C; and one final cycle of 7 min at 72°C. The resulting PCR product was analysed on an agarose gel and isolated using QIAquick PCR purification Kit (Cat No.: 218104). The isolation was performed according to manufacture's protocol. The PCR product was dissolved in 50 µl H2O and cloned into pDonr201 (Cat. No.: 11798-014) using Gateway™ BP clonase enzyme mix (Cat. No.: 11789-013). After ligation, the plasmid was used to transform the electrocompetent *E*. *coli* strain DH10B. Individual clones were sequenced using DonorF primer (5'- TCG CGT TAA CGC TAG CAT GGA TCT C-3') and DonorR primer (5'- GTA ACA TCA GAG ATT TTG AGA CAC-3') and obtained sequences were analysed for identity or similarity using the standard nucleotide-nucleotide BLAST (blastn) similarity search program and the sequences of GenBank+EMBL+DDBJ+PDB (but no EST, STS, GSS, or phase 0, 1 or 2 HTGS sequences). All eight clones displayed 100 % identity with *homo sapiens* membrane cofactor protein (CD46, trophoblast-lymphocyte cross-reactive antigen).

The full-length human CD46 protein is encoded by 14 exons with alternative splicing giving rise to a number of variants. The 8 clones found in this screen were composed of 4 different variants. Clones CY_03, CY_17, CY_19, and CY_46 lack exon 7 (encoding STP A), exon 8 (encoding STP B), and exon 13 (encoding CYT A). Clone CY_11 lacks exons 7 and 8, clone CY_33 lacks exons 7 and 9, and clones CY_28 and CY_54 lack only exon 7.

### Example 4.

### Construction, expression, and purification of a CD46-Fc fusion protein.

A synthetic construct is produced allowing for the expression of an bispecific adaptor protein, carrying an N-terminal human CD46 extracellular domain (ECD) fused to a C-terminal human Fc-γ3 domain. Thus, the CD46 ECD coding region (nucleotides 34 to 1120) is PCR amplified from clone CY_28 using the primers 46ECD-F (5'-GAG GAG GAG CAG CTG GCC ACC ATG GAG CCT CCC GGC CGC CGC-3') and 46ECD-B (5'-GAG GAG GAG AAG CTT AAC ATC CAA ACT GTC AAG TAT TC-3'), digested with the restriction endonucleases Pvu2 and Hind3, and cloned into the expression vector pCEP-Fc-C. This vector is a derivative of the episomal mammalian expression vector pCEP4 (Invitrogen), carrying the Epstein-Barr Virus replication origin (oriP) and nuclear antigen (encoded by the EBNA-1 gene) to permit extrachromosomal replication, and contains a Puromycin selection marker in place of the original Hygromycin B resistance gene. The resulting plasmid, pCEP/46-Fc, drives expression of a CD46 ECD - Fc domain fusion protein under the control of a CMV promoter.

Expression of the fusion construct is done in HEK-293T cells. One day before transfection, 5x10⁶ 293T cells are plated onto a 10cm tissue culture plate. Cells are then transfected with pCEP/46-Fc using Lipofectamin Plus (Invitrogen), incubated one day, and subjected to selection in the presence of 10 µg/ml puromycin. After 24 hours of selection, puromycin-resistant cells are transfered to a Poly-L-Lysine coated 15 cm tissue culture plate and grown to confluency. Medium is replaced by serum-free medium and supernatant containing the CD46-Fc fusion protein is collected every 3 days. Pooled supernatants are filtered through a 0.22 µM Millex GV sterile filter (Millipore) and applied to a protein A-sepharose column. The column is washed with 5 column volumes of 20 mM Tris pH 8.0, 150 mM NaCl, and bound protein is eluted with citrate-phosphate buffer pH 3.6. 1 ml fractions are collected in tubes containing 0.1 ml of 0.5 M Na₂HPO₄ for neutralization. Positive fractions are identified by SDS-PAGE and pooled. The buffer is exchanged with phosphate-buffered saline (PBS) by ultrafiltration through Ultrafree Biomax 10k (Millipore). The purified protein in PBS is then filtered through 0.22 µM Millex GV sterile filters (Millipore) and stored at 4°C.

### Example 5.

### Construction, expression, and purification of CD46-scFv fusion proteins.

Synthetic constructs are produced allowing for the expression of bispecific adaptor proteins. Each carries an N-terminal human CD46 extracellular domain (ECD), a central mouse single-chain antibody, specific for either human ErbB-2 (scFv-FRP5) or human EGFR (scFv-225), as well as a C-terminal FLAG epitope tag. Thus, the CD46 ECD coding region (nucleotides 34 to 1120) is PCR amplified from clone CY_28 using the primers 46ECD-F2 (5'-GAG GAG GAG GGT ACC GCC ACC ATG GAG CCT CCC GGC CGC CGC-3') and 46ECD-B2 (5'-GAG GAG GAG GCG GCC GCC AAC ATC CAA ACT GTC AAG TAT TC-3'). The scFv-FRP5 coding region is PCR amplified from the plasmid pWW52 (Wels et al., 1992) with the primers FRP5-F (5'-GAG GAG GAG GGC GGC CGC TCT CAG GTA CAA CTG CAG CAG TCT GG-3') and FRP5-B (5'-GAG GAG GAG CTC GAG GAT CTC CAA TTT TGT CCC CGA GCC G-3'). The scFv-225 coding region is PCR amplified from pSW202-225 (Wels et al., 1995) with the primers 225-F (GAG GAG GAG GGC GGC CGC CTT CAG GTA CAA CTG CAG GAG TCA GG) and 225-B (GAG GAG GAG CTC GAG GAT CTC CAG CTT GGT CCC AGC ACC G). The CD45 ECD PCR product is digested with the restriction endonucleases Kpn1 and Not1, and the scFv PCR products with the restriction endonucleases Not1 and Xho1. The CD46 ECD fragment is then ligated into pCEP-FLAG-C previously digested with the restriction endonucleases Kpn1 and Xho1, together with either the scFv-FRP5 or the scFv-225 fragment, yielding the expression vectors pCEP/CD46-FRP5-FLAG and pCEP/CD46-225-FLAG. The pCEP-FLAG-C vector is a derivative of the episomal mammalian expression vector pCEP4 (Invitrogen), carrying the Epstein-Barr Virus replication origin (oriP) and nuclear antigen (encoded by the EBNA-1 gene) to permit extrachromosomal replication, and contains a Puromycin selection marker in place of the original Hygromycin B resistance gene. The plasmids pCEP/CD46-FRP5-FLAG and pCEP/CD46-225-FLAG drive expression of CD46ECD-scFv(FRP5)-FLAG and CD46ECD-scFv(225)-FLAG fusion protein under the control of a CMV promoter, respectively.

Expression of the fusion constructs is done in HEK-293T cells. One day before transfection, 5x10⁶ 293T cells are plated onto a 10cm tissue culture plate. Cells are then transfected with pCEP/CD46-FRP5-FLAG or pCEP/CD46-225-FLAG using Lipofectamin Plus (Invitrogen), incubated one day, and subjected to selection in the presence of 10 µg/ml puromycin. After 24 hours of selection, puromycin-resistant cells are transfered to a Poly-L-Lysine coated 15 cm tissue culture plate and grown to confluency. Medium is replaced by serum-free medium and supernatant containing the respective CD46ECD fusion protein is collected every 3 days and pooled. Cellular debris is removed by centrifugation at 3000 g for 10 minutes followed by filtration through a 0.2µm filter.

Supernatant containing the respective FLAG tagged protein is equilibrated to pH 8.0 with 20 mM HEPES and 40 % w/v ammonium sulphate is added to the supernatant. Ammonium sulphate precipitation is performed at 4°C with stirring for 6-8 h. The precipitates are then pelleted for 1h at 8000 g at 4°C. Each of the pellets is resuspended in 20 mM HEPES, pH 8.0 supplemented with 50 mM NaCl. Insoluble material is removed by filtration through a 0.2 µm filter.

The CD46ECD-scFv(FRP5)-FLAG and CD46ECD-scFv(225)-FLAG fusion proteins are purified by affinity chromatography using M2-FLAG-Agarose resin (Sigma, Cat. No. A220) as follows. Each of the concentrated samples is applied to a FLAG column at room temperature using a peristaltic pump at flow rates of 1-2 ml/ min. The columns are then washed with at least 20 column volumes of TBS (10 mM Tris/HCl pH 7.5). Bound proteins are eluted with FLAG peptide (Sigma, Cat. No. F3290) at a concentration of 0.1 mg/ml in TBS. The protein containing fractions are pooled and concentrated using Millipore Ultrafree centrifugal filters 5K (Millipore, Cat. No.UFV4BCC25). The same concentration filters are used to perform a buffer exchange to PBS and to get rid of residual FLAG peptide. The purified protein preparations are sterile filtered using Millipore Millex filters (Millipore Cat. No. SLGV 004 SL) and stored in PBS at 4°C.

### Example 6.

### Redirecting Ad3 to Fc receptor-positive cells by using CD46-Fc receptor fusion protein

To test whether the CD46-Fc fusion protein allows for CD46-independent infection of cells via Fc receptor, a derivative of the Ad3-refractory cell line BHK is constructed. Thus, the cDNA encoding human high affinity Fcγ receptor I (CD64) is amplified from human spleen polyA+ RNA (Clontech, #6542-1) by RT-PCR, cloned into pcDNA3.1 (Invitrogen), and transfected into subconfluent BHK cells. After 10 days of selection in G-418 (1 mg/ml; Invitrogen) FcR-positive cells are isolated using a FACS Vantage fluorescence activated cell sorter (Becton Dickinson), yielding the cell line BHK/FcR.

For infectivity studies, a recombinant Ad5 virus carriing a chimeric fiber with the receptor binding knob domain of the Ad3 fiber is used. Such a virus no longer binds the coxsackie-adenovirus receptor (CAR) protein normally used by Ad5, but instead infects cells via binding to the Ad3 receptor (Von Seggern et al., 2000). Thus, a fiber gene-deleted Ad5 virus carriing an eGFP marker gene is produced in 293 cells expressing the chimeric fiber protein (He et al., 1998). Intact virus particles are isolated by purification over a CsCl gradient followed by dialysis against PBS, yielding pure adenovirus preparations with titers of 10¹² pfu/ml.

To demonstrate that CD46-Fc fusion protein enables the Ad5/Ad3 chimeric virus to infect BHK/FcR cells, the following experiments are carried out. BHK/FcR cells (or, as a negative control, parental BHK cells) are incubated with Ad5/Ad3-eGFP at an MOI of 10, premixed with 1 µg of either the CD46-Fc protein, or an unspecific Fc fusion protein. 48 hours post infections, eGFP staining is analyzed by flow cytometry.

### Example 7.

### Redirecting Ad3 to ErbB-2 or EGFR overexpressing human tumor cells by using CD46ECD-scFv(FRP5) or CD46ECD-scFv(225) fusion proteins

Adenovirus has the capability to infect essentially all tissues and cell types. To prevent nonspecific infection in a therapeutic setting, it is crucial that virus preparations are prebound to the chimeric adapter protein under saturating conditions. For this purpose, purified preparations of the eGFP-encoding chimeric Ad5/3 virus described in Example 6 are used. Thus, 10¹² pfu purified Ad5/3 are incubated with 10µg CD46ECD-scFv-FLAG fusion protein (a 100-fold molar excess of protein over virion, or approximately 8 molecules of fusion protein per fiber) for 2 hours at 4°C in PBS. Unbound adapter protein is then removed by ultracentrifugation through Centricon YM-100 (MW cut-off 100'000 Da).

The ability of CD46ECD-FRP5 coated Ad5/3 virions to infect ErbB-2-positive cells is assessed by *in vitro* infection of human breast cancer cells expressing various levels of ErbB-2. Thus, SKBR3 (high ErbB-2), BT474 (high ErbB-2), T47D (low ErbB-2), and MDA MB 468 (ErbB-2 negative) are infected at MOIs of 10 or 100. A similar experiment is carried out to assess the ability of CD46ECD-225 coated Ad5/3 virions to infect EGFR-positive cells. For this purpose, the human tumor cell lines A431 (high EGFR), MCF7 (low EGFR), and SW620 (EGFR-negative) are infected at MOIs of 10 or 100. As negative controls, mouse NIH/3T3 fibroblasts, which express ErbB-2 and EGFR but do not bind scFv-FRP5 or scFv-225, are also infected. Success of the infections is evaluated by flow cytometric analysis. To show that each of the cell lines is in principle infectable, identical infections are carried out in parallel with Ad5/3 virions not coated with fusion proteins.

To investigate whether CD46ECD-scFv coated Ad5/3 virions can infect tumor cells *in vivo*, 10⁶ ErbB-2 overexpressing BT464 cells are implanted subcutaneously into nude mice. When tumor size reaches about 5 mm in diameter, animals are treated by intratumoral injection of CD46ECD-FRP5 coated Ad5/3 virions at 10¹⁰ pfu/animal. In the same manner, EGFR overexpressing A431 cells are implanted into nude mice and infected with CD46ECD-225 coated Ad5/3 virions. Three days later, animals are sacrificed and tumors removed for analysis of GFP expression. To demonstrate selectivity of infection, tissue surrounding the tumor mass is also removed and analyzed.

**Table 1**

| **Clone** | SCR1 | SCR2 | SCR3 | SCR4 | STP A | STP B | STP C | UK | TM A | TM B | CYT A | CYT B |
|---|---|---|---|---|---|---|---|---|---|---|---|---|
| **Exon** | 2 | 3/4 | 5* | 6 | 7 | 8 | 9 | 10 | 11 | 12 | 13 | 14 |
| **CY_03** | √ | √ | √ | √ | | | √ | √ | √ | √ | | √ |
| **CY_11** | √ | √ | √ | √ | | | √ | √ | √ | √ | √ | √* |
| **CY_17** | √ | √ | √ | √ | | | √ | √ | √ | √ | | √ |
| **CY_19** | √ | √ | √ | √ | | | √ | √ | √ | √ | | √ |
| **CY_28** | √ | √ | √ | √ | | √ | √ | √ | √ | √ | √ | √* |
| **CY_33** | √ | √ | √ | √ | | √ | | √ | √ | √ | √ | √* |
| **CY_46** | √ | √ | √ | √ | | | √ | √ | √ | √** | | √ |
| **CY_54** | √ | √ | √ | √ | | √ | √ | √ | √ | √ | √ | √* |

### REFERENCES

Boorsma M, Hoenke S, Marrero A, Fischer R, Bailey JE, Renner WA, Bachmann MF. (2002) Bioprocess applications of a Sindbis virus-based temperature-inducible expression system. Biotechnol. Bioeng. 79, 602-609.

Bredenbeek PJ, Frolov I, Rice CM, and Schlesinger S. (1993) Sindbis virus expression vectors: packaging of RNA replicons by using defective helper RNAs. *J Virol.* **67**, 6439-46.

Breyer B, Jiang W, Cheng H, Zhou L, Paul R, Feng T, He TC. (2001) Adenoviral vector-mediated gene transfer for human gene therapy. *Curr Gene Ther.* 1(2):149-62.

Bronte V.Genetic vaccination for the active immunotherapy of cancer. (2001) *Curr Gene Ther.* 1(1):53-100.

Davies J, Riechmann L. (1996) Single antibody domains as small recognition units: design and in vitro antigen selection of camelized, human VH domains with improved protein stability. Protein Eng. 9, 531-537.

Defer C, Belin MT, Caillet-Boudin ML, Boulanger P. (1990) Human adenovirus-host cell interactions: comparative study with members of subgroups B and C. *J Virol.* **64**, 3661-73.

Fivash M, Towler EM, Fisher RJ. (1998) BIAcore for macromolecular interaction. Curr. Opin. Biotechnol. 9, 97-101.

He TC, Zhou S, Da Costa LT, Yu J, Kinzler KW, Vogelstein B (1998) A simplified system for generating recombinant adenoviruses. *Proc. Natl. Acad. Sci. USA* **95**, 2509-2514.

Holliger P, Prospero T, Winter G. (1993) "Diabodies": small bivalent and bispecific antibody fragments. Proc. Natl. Acad. Sci. *U S A* 90, 6444-6448.

Horwitz, MS. (1996) Adenoviruses. In *Virology, 3*^{*rd*} *edition* (editors B. N. Fields, D. M. Knipe, and P. M. Howley), 2149-2171. Lippincott Williams & Wilkins.

Kabat EA, Wu TT. (1991) Identical V region amino acid sequences and segments of sequences in antibodies of different specificities. Relative contributions of VH and VL genes, minigenes, and complementarity-determining regions to binding of antibody-combining sites. J. Immunol. 147, 1709-1719.

Kawakami Y, Li H, Lam JT, Krasnykh V, Curiel DT, Blackwell JL. (2003) Substitution of the adenovirus serotype 5 knob with a serotype 3 knob enhances multiple steps in virus replication. *Cancer Res* **63**, 1262-1269.

Loser P, Huser A, Hillgenberg M, Kumin D, Both GW, Hofmann C. (2002) Advances in the development of non-human viral DNA-vectors for gene delivery. *Curr Gene Ther.* 2(2):161-71.

Martin AB, Webber S, Fricker FJ, Jaffe R, Demmler G, Kearney D, Zhang YH, Bodurtha J, Gelb B, Ni J, et al. (1994) Acute myocarditis. Rapid diagnosis by PCR in children. *Circulation* **90**, 330-339.

Mayr GA, and Freimuth P. (1997) A single locus on human chromosome 21 directs the expression of a receptor for adenovirus type 2 in mouse A9 cells. *J Virol.* **71**, 412-8.

Macleod K. (2000) Tumor suppressor genes. Curr. Opin. Genet. Dev. 10, 81-93.

Milenic DE. (2002) Monoclonal antibody-based therapy strategies: providing options for the cancer patient. Curr. Pharm. Des. 8, 1749-1764.

Mitani K, Kubo S. Adenovirus as an integrating vector. (2002) *Curr Gene Ther.* 2(2):135-44.

Munger K. (2002) Disruption of oncogene/tumor suppressor networks during human carcinogenesis. Cancer Invest. 20, 71-81.

Ochiya T, Nagahara S, Sano A, Itoh H, Terada M. (2001) Biomaterials for gene delivery: atelocollagen-mediated controlled release of molecular medicines. *Curr Gene Ther. 1*(1):31-52.

Penichet ML, Morrison SL. (2001) Antibody-cytokine fusion proteins for the therapy of cancer. J. Immunol. Methods. 248, 91-101.

Purcell DF, Russell SM, Deacon NJ, Brown MA, Hooker DJ, McKenzie IF. (1991) Alternatively spliced RNAs encode several isoforms of CD46 (MCP), a regulator of complement activation. Immunogenetics 33, 335-344.

Russell SM, Sparrow RL, McKenzie IF, Purcell DF. (1992) Tissue-specific and allelic expression of the complement regulator CD46 is controlled by alternative splicing. Eur J Immunol. 22, 1513-1518.

Schlesinger S. (1993) Alphaviruses-vectors for the expression of heterologous genes. Trends Biotechnol. 11, 18-22.

Shenk T. (1996) Adenoviridae: the viruses and their replication. In *Virology, 3*^{*rd*} *edition* (editors B. N. Fields, D. M. Knipe, and P. M. Howley), 2111-2148. Lippincott Williams & Wilkins.

Stevenson SC, Rollence M, White B, Weaver L, and McClelland A. (1995) Human adenovirus serotypes 3 and 5 bind to two different cellular receptors via the fiber head domain. *J*. *Virol.* **69**, 2850-2857

Teh BT, Larsson C, Nordenskjold M. (1999) Tumor suppressor genes (TSG). Anticancer Res. 19, 4715-4728.

Van den Driessche T, Collen D, Chuah MK. (2001)Viral vector-mediated gene therapy for hemophilia. *Curr Gene Ther.* 1(3):301-15

Von Seggern DJ, Huang S, Fleck SK, Stevenson SC, Nemerow GR. (2000) Adenovirus vector pseudotyping in fiber-expressing cell lines: improved transduction of Epstein-Barr virus-transformed B cells. *J*. *Virol.* **74**, 354-62.

Weiner LM. (1999) Monoclonal antibody therapy of cancer. Semin. Oncol. 26 (5 Suppl 14), 43-51.

Weiner LM. (1999) An overview of monoclonal antibody therapy of cancer. Semin. Oncol. 26 (4 Suppl 12), 41-50.

Wels W, Harwerth IM, Zwickl M, Hardman N, Groner B, Hynes NE. Construction, bacterial expression and characterization of a bifunctional single-chain antibody-phosphatase fusion protein targeted to the human erbB-2 receptor. Biotechnology (N Y). 1992 Oct;10(10):1128-32.

Wels W, Beerli R, Hellmann P, Schmidt M, Marte BM, Kornilova ES, Hekele A, Mendelsohn J, Groner B, Hynes NE. EGF receptor and p185erbB-2-specific single-chain antibody toxins differ in their cell-killing activity on tumor cells expressing both receptor proteins. Int J Cancer. 1995 Jan 3;60(1):137-44.

Wickham TJ, Mathias P, Cheresh DA, Nemerow GR. (1993) Integrins alpha v beta 3 and alpha v beta 5 promote adenovirus internalization but not virus attachment. *Cell* **73**, 309-19

Withoff S, Helfrich W, de Leij LF, Molema G. (2001) Bi-specific antibody therapy for the treatment of cancer. Curr. Opin. Mol. Ther. 3, 53-62.

Wu Q, Moyana T, Xiang J. (2001) Cancer gene therapy by adenovirus-mediated gene transfer. *Curr Gene Ther.* 1(1):101-22.

Zhu YY, Machleder EM, Chenchik A, Li R, and Siebert PD. (2001) Reverse transcriptase template switching: a SMART approach for full-length cDNA library construction. *Biotechniques* **30**, 892-7.

## Claims

1. A modified polypeptide comprising
(a) a polypeptide derived from the extracellular domain of CD46, and
(b) a component capable of binding to a cell surface molecule, with the provisio that (b) is not a polypeptide derived from CD55.

2. The modified polypeptide of claim 1, wherein the polypeptide (a) does not comprise the wildtype STP-A region of CD46.

3. The modified polypeptide of claims 1 and 2, wherein the polypeptide (a) comprises at least all four SCR-regions of CD46, and preferably also comprises the regions STP-B and STP-C of CD46.

4. The modified polypeptide of claims 1 to 3, wherein the polypeptide (a) is encoded by a nucleic acid comprising
(i) a nucleic acid sequence as defined in the SEQ IDs No. 12, 14 or 16,
(ii) a nucleic acid sequence which hybridizes to the nucleic acid sequence as defined in (i) under stringent conditions,
(iii) a nucleic acid sequence which is degenerate as a result of the genetic code to the nucleic acid sequence as defined in (i) and (ii) and which encodes a polypeptide having essentially the same binding activity as the extracellular domain of CD46, or
(iv) a nucleic acid sequence having a sequence identity of at least 70% with the nucleic acid sequence as defined in (i), or a fragment thereof, and which encodes a polypeptide having essentially the same binding activity as the extracellular domain of CD46.

5. The modified polypeptide of claim 1, wherein the polypeptide (a) is defined as in the amino acid sequence according to SEQ IDs No. 13, 15 or 17.

6. The modified polypeptide of claims 1 to 5, wherein the component (b) is selected from the group consisting of a small organic molecule, a peptide, and a polypeptide.

7. The modified polypeptide of claims 1 to 6, wherein component (b) is not a polypeptide derived from a polypeptide of the complement pathway.

8. The modified polypeptide of claim 6, wherein the small organic molecule is selected from the group consisting of a non-proteinaceous hormone, a neurotransmitter and a synthetic molecule capable of binding to a surface receptor.

9. The modified polypeptide of claim 6, wherein the component (b) is capable of specific binding to a surface receptor with a dissociation constant of lower than 1 µM.

10. The modified polypeptide of any of claims 1 to 9, wherein the component (b) is capable of binding a molecule selected from the group consisting of a cell type-specific cell surface molecule, a disorder-specific cell surface molecule, a cell-surface receptor, a cell-adhesion molecule and a sugar moiety located on one of the aforementioned molecules, in particular wherein the component (b) is capable of binding a molecule selected from the group consisting of a leukocyte antigen, a receptor tyrosine kinase, a receptor of the TNF receptor family, a cytokine receptor, a G-protein-coupled-receptor, a receptor tyrosine phosphatase, a chemokine receptor, a scavenger receptor, a Fc-receptor, a tetraspannin, a member of the Ig-superfamily and a lectin.

11. The modified polypeptide of claim 10, wherein the component (b) is an antibody or an antibody fragment.

12. The modified polypeptide of claim 11, wherein the antibody fragment is selected from the group consisting of an scFab, Fab, F(ab')₂, diabodies, and an scFv.

13. The modified polypeptide of claim 6, wherein the polypeptide of (b) is selected from the group consisting of a ligand of cell type-specific cell surface molecule, a ligand of a disorder-specific cell surface molecule, a ligand of a cell-surface receptor, a ligand of a cell-adhesion molecule and a ligand of a sugar moiety located on one of the aforementioned molecules, in particular wherein component (b) is selected from the group consisting of a ligand of a leukocyte antigen, a ligand of a receptor tyrosine kinase, a ligand of a receptor of the TNF receptor family, a ligand of a cytokine receptor, a ligand of a G-protein-coupled-receptor, a ligand of a receptor tyrosine phosphatase, a ligand of a chemokine receptor, a ligand of a scavenger receptor, a ligand of a Fc-receptor, a ligand of a tetraspannin, a ligand of a member of the Ig-superfamily and a ligand of a lectin.

14. The modified polypeptide of any one of claims 1 to 13, wherein the polypeptide of (a) and the component (b) are linked to each other by a covalent linkage, preferably chemical crosslinking or genetic fusion.

15. The modified polypeptide of any one of claims 1 to 14, wherein the polypeptide of (a) and the component (b) are crosslinked via a spacer, wherein the spacer is selected from the group consisting of heterobifunctional cross-linkers, flexible amino acid linkers, like the hinge regions of Immunoglobulins, glycine serine linkers and glycine linkers, homobifunctional cross-linkers and stable ligand-receptor pairs, like for example the biotin-streptavidin system.

16. The modified polypeptide of one of claims 1 to 14, wherein the modified polypeptide is defined as in the amino acid sequence according to SEQ IDs No. 19 or 21.

17. A nucleic acid comprising a nucleic acid sequence encoding a modified polypeptide according to one of claims 1 to 16.

18. The nucleic acid of claim 17, wherein the nucleic acid comprises
(i) a nucleic acid sequence as defined in the SEQ IDs No. 12, 14 or 16,
(ii) a nucleic acid sequence which hybridizes to the nucleic acid sequence as defined in (i) under stringent conditions,
(iii) a nucleic acid sequence which is degenerate as a result of the genetic code to the nucleic acid sequence as defined in (i) and (ii) and which encodes a polypeptide having essentially the same binding activity as the extracellular domain of CD46, or
(iv) a nucleic acid sequence having a sequence identity of at least 70% with the nucleic acid sequence as defined in (i), or a fragment thereof, and which encodes a polypeptide having essentially the same binding activity as the extracellular domain of CD46.

19. A recombinant expression vector comprising the nucleic acid according to claim 17 or 18 operably linked to at least one regulatory sequence for expression of the modified protein.

20. A host cell containing a nucleic acid according to claims 17 to 18 or a vector according to claim 19.

21. The host cell of claim 20, wherein the host cell is a cell selected from the group consisting of a monocellular phagocyte lineage cell, 293 cells, BHK cells and Sf9 cells.

22. A method of producing a modified polypeptide according to one of claims 1 to 16, comprising culturing a host cell according to claim 20 or 21 under conditions suitable for expression of the modified polypeptide in the host cell and isolating the modified polypeptide from the host cell.

23. The method of claim 22, wherein the isolated modified polypeptide is further formulated as a pharmaceutical composition.

24. A method for preventing or treating a patient in need of such treatment, wherein the patient is administered a therapeutically effective amount of a modified polypeptide according to one of claims 1 to 16.

25. A modified polypeptide according to one of claims 1 to 16 for use in medicine.

26. A pharmaceutical composition comprising a modified polypeptide according to one of claims 1 to 16 and a pharmaceutically acceptable carrier.

27. A pharmaceutical composition according to claim 26, further comprising an adenovirus of the subtype B or a virus, expressing a molecule capable of binding a CD46 extracellular domain, which adenovirus or virus has been genetically engineered.

28. The pharmaceutical composition of claim 27, wherein the adenovirus or virus has been genetically engineered by introducing a therapeutically active gene construct.

29. The pharmaceutical composition of claim 28, wherein the therapeutically active gene construct comprises a therapeutically active gene operably linked to at least one regulatory sequence for expression of the therapeutically active gene.

30. The pharmaceutical composition of claim 29, wherein the therapeutically active gene is a tumor supressor gene, for example selected from the group consisting of p53, Retinoblastoma, NF2, BRCA1, BRCA2, MSH2, MSH6, MLH1, CDKN2, Apafl, DPC4, PKD1, HPC1 and VHL.

31. Use of
a modified polypeptide according to one of claims 1 to 16 and an adenovirus of the subtype B or a virus, expressing a molecule capable of binding a CD46 extracellular domain, which adenovirus or virus has been genetically engineered,
or use of a pharmaceutical composition according to one of claims 27 to 30
in the manufacture of a pharmaceutical for the treatment of a disorder or a disease selected from the group consisting of SCID, cystic fibrosis, arthritis, multiple sclerosis and cancer, in particular cancer caused by cells deficient in any one tumor suppressor gene.


SEQUENCE LISTING

<110>  Cytos Biotechnology AG

<120>  Polypeptides involved in cell-entry of the Adenoviruses of Subtype B

<130>  C62115EP

<160>  21

<170>  PatentIn version 3.1

<210>  1
<211>  1134
<212>  DNA
<213>  homo sapiens

<400>  1
atggagcctc ccggccgccg cgagtgtccc tttccttcct ggcgctttcc tgggttgctt      60
ctggcggcca tggtgttgct gctgtactcc ttctccgatg cctgtgagga gccaccaaca     120
tttgaagcta tggagctcat tggtaaacca aaaccctact atgagattgg tgaacgagta     180
gattataagt gtaaaaaagg atacttctat atacctcctc ttgccaccca tactatttgt     240
gatcggaatc atacatggct acctgtctca gatgacgcct gttatagaga aacatgtcca     300
tatatacggg atcctttaaa tggccaagca gtccctgcaa atgggactta cgagtttggt     360
tatcagatgc actttatttg taatgagggt tattacttaa ttggtgaaga aattctatat     420
tgtgaactta aaggatcagt agcaatttgg agcggtaagc ccccaatatg tgaaaaggtt     480
ttgtgtacac cacctccaaa aataaaaaat ggaaaacaca cctttagtga agtagaagta     540
tttgagtatc ttgatgcagt aacttatagt tgtgatcctg cacctggacc agatccattt     600
tcacttattg gagagagcac gatttattgt ggtgacaatt cagtgtggag tcgtgctgct     660
ccagagtgta aagtggtcaa atgtcgattt ccagtagtcg aaaatggaaa acagatatca     720
ggatttggaa aaaaatttta ctacaaagca acagttatgt ttgaatgcga taagggtttt     780
tacctcgatg gcagcgacac aattgtctgt gacagtaaca gtacttggga tcccccagtt     840

ccaaagtgtc ttaaagtgtc gacttcttcc actacaaaat ctccagcgtc cagtgcctca 900

ggtcctaggc ctacttacaa gcctccagtc tcaaattatc caggatatcc taaacctgag 960

gaaggaatac ttgacagttt ggatgtttgg gtcattgctg tgattgttat tgccatagtt 1020

gttggagttg cagtaatttg tgttgtcccg tacagatatc ttcaaaggag gaagaagaaa 1080

ggcacatacc taactgatga gacccacaga gaagtaaaat ttacttctct ctga 1134

<210> 2

<211> 377

<212> PRT

<213> homo sapiens

<220>

<221> SIGNAL

<222> (1)..(34)

<223>

<220>

<221> MISC_FEATURE

<222> (35)..(328)

<223> Extracellular domain

<220>

<221> TRANSMEM

<222> (329)..(351)

<223>

<220>

<221> MISC_FEATURE

<222> (35)..(95)

<223> SCR-1 domain

<220>

<221>  MISC_FEATURE
<222>  (98)..(158)
<223>  SCR-2 domain

<220>
<221>  MISC_FEATURE
<222>  (161)..(224)
<223>  SCR-3 domain

<220>
<221>  MISC_FEATURE
<222>  (227)..(284)
<223>  SCR-4 domain

<220>
<221>  MISC_FEATURE
<222>  (286)..(300)
<223>  STP-B region

<220>
<221>  MISC_FEATURE
<222>  (301)..(314)
<223>  STP-C region

<400>  2

Met Glu Pro Pro Gly Arg Arg Glu Cys Pro Phe Pro Ser Trp Arg Phe
1               5                   10                  15

Pro Gly Leu Leu Leu Ala Ala Met Val Leu Leu Leu Tyr Ser Phe Ser
            20                  25                  30

Asp Ala Cys Glu Glu Pro Pro Thr Phe Glu Ala Met Glu Leu Ile Gly
        35                  40                  45

Lys Pro Lys Pro Tyr Tyr Glu Ile Gly Glu Arg Val Asp Tyr Lys Cys

50                  55                  60

Lys Lys Gly Tyr Phe Tyr Ile Pro Pro Leu Ala Thr His Thr Ile Cys
65                  70                  75                  80

Asp Arg Asn His Thr Trp Leu Pro Val Ser Asp Asp Ala Cys Tyr Arg
                85                  90                  95

Glu Thr Cys Pro Tyr Ile Arg Asp Pro Leu Asn Gly Gln Ala Val Pro
            100                 105                 110

Ala Asn Gly Thr Tyr Glu Phe Gly Tyr Gln Met His Phe Ile Cys Asn
        115                 120                 125

Glu Gly Tyr Tyr Leu Ile Gly Glu Glu Ile Leu Tyr Cys Glu Leu Lys
    130                 135                 140

Gly Ser Val Ala Ile Trp Ser Gly Lys Pro Pro Ile Cys Glu Lys Val
145                 150                 155                 160

Leu Cys Thr Pro Pro Pro Lys Ile Lys Asn Gly Lys His Thr Phe Ser
                165                 170                 175

Glu Val Glu Val Phe Glu Tyr Leu Asp Ala Val Thr Tyr Ser Cys Asp
            180                 185                 190

Pro Ala Pro Gly Pro Asp Pro Phe Ser Leu Ile Gly Glu Ser Thr Ile
        195                 200                 205

Tyr Cys Gly Asp Asn Ser Val Trp Ser Arg Ala Ala Pro Glu Cys Lys
    210                 215                 220

Val Val Lys Cys Arg Phe Pro Val Val Glu Asn Gly Lys Gln Ile Ser
225                 230                 235                 240

Gly Phe Gly Lys Lys Phe Tyr Tyr Lys Ala Thr Val Met Phe Glu Cys
                245                 250                 255

Asp Lys Gly Phe Tyr Leu Asp Gly Ser Asp Thr Ile Val Cys Asp Ser
            260                 265                 270

Asn Ser Thr Trp Asp Pro Pro Val Pro Lys Cys Leu Lys Val Ser Thr
        275                 280                 285

Ser Ser Thr Thr Lys Ser Pro Ala Ser Ser Ala Ser Gly Pro Arg Pro
    290                 295                 300

Thr Tyr Lys Pro Pro Val Ser Asn Tyr Pro Gly Tyr Pro Lys Pro Glu
305                 310                 315                 320

Glu Gly Ile Leu Asp Ser Leu Asp Val Trp Val Ile Ala Val Ile Val
                325                 330                 335

Ile Ala Ile Val Val Gly Val Ala Val Ile Cys Val Val Pro Tyr Arg
            340                 345                 350

Tyr Leu Gln Arg Arg Lys Lys Lys Gly Thr Tyr Leu Thr Asp Glu Thr
        355                 360                 365

His Arg Glu Val Lys Phe Thr Ser Leu
    370                 375

<210>  3

<211>  15

<212>  PRT

<213>  artificial sequence

<220>

<223>  polypeptide sequence of the STP-A region, which is inserted befor
       e the STP-B region of CD46 in certain splice variants of CD46

<400>  3

Val Leu Pro Pro Ser Ser Thr Lys Pro Pro Ala Leu Ser His Ser
1               5                   10                  15

<210>  4

<211>  1110

<212>  DNA

<213>  homo sapiens

<400>  4
atggagcctc ccggccgccg cgagtgtccc tttccttcct ggcgctttcc tgggttgctt      60

ctggcggcca tggtgttgct gctgtactcc ttctccgatg cctgtgagga gccaccaaca     120

tttgaagcta tggagctcat tggtaaacca aaaccctact atgagattgg tgaacgagta     180

gattataagt gtaaaaaagg atacttctat atacctcctc ttgccaccca tactatttgt     240

gatcggaatc atacatggct acctgtctca gatgacgcct gttatagaga aacatgtcca     300

tatatacggg atcctttaaa tggccaagca gtccctgcaa atgggactta cgagtttggt     360

tatcagatgc actttatttg taatgagggt tattacttaa ttggtgaaga aattctatat 420

tgtgaactta aaggatcagt agcaatttgg agcggtaagc ccccaatatg tgaaaaggtt 480

ttgtgtacac cacctccaaa aataaaaaat ggaaaacaca cctttagtga agtagaagta 540

tttgagtatc ttgatgcagt aacttatagt tgtgatcctg cacctggacc agatccattt 600

tcacttattg gagagagcac gatttattgt ggtgacaatt cagtgtggag tcgtgctgct 660

ccagagtgta aagtggtcaa atgtcgattt ccagtagtcg aaaatggaaa acagatatca 720

ggatttggaa aaaaatttta ctacaaagca acagttatgt ttgaatgcga taagggtttt 780

tacctcgatg gcagcgacac aattgtctgt gacagtaaca gtacttggga tcccccagtt 840

ccaaagtgtc ttaaaggtcc taggcctact tacaagcctc cagtctcaaa ttatccagga 900

tatcctaaac ctgaggaagg aatacttgac agtttggatg tttgggtcat tgctgtgatt 960

gttattgcca tagttgttgg agttgcagta atttgtgttg tcccgtacag atatcttcaa 1020

aggaggaaga agaaagggaa agcagatggt ggagctgaat atgccactta ccagactaaa 1080

tcaaccactc cagcagagca gagaggctga 1110

<210> 5

<211> 1089

<212> DNA

<213> homo sapiens

<400> 5
atggagcctc ccggccgccg cgagtgtccc tttccttcct ggcgctttcc tgggttgctt 60

ctggcggcca tggtgttgct gctgtactcc ttctccgatg cctgtgagga gccaccaaca 120

tttgaagcta tggagctcat tggtaaacca aaaccctact atgagattgg tgaacgagta 180

gattataagt gtaaaaaagg atacttctat atacctcctc ttgccaccca tactatttgt 240

gatcggaatc atacatggct acctgtctca gatgacgcct gttatagaga aacatgtcca 300

tatatacggg atcctttaaa tggccaagca gtccctgcaa atgggactta cgagtttggt 360

tatcagatgc actttatttg taatgagggt tattacttaa ttggtgaaga aattctatat 420

tgtgaactta aaggatcagt agcaatttgg agcggtaagc ccccaatatg tgaaaaggtt 480

ttgtgtacac cacctccaaa aataaaaaat ggaaaacaca cctttagtga agtagaagta 540

tttgagtatc ttgatgcagt aacttatagt tgtgatcctg cacctggacc agatccattt 600

tcacttattg gagagagcac gatttattgt ggtgacaatt cagtgtggag tcgtgctgct 660

ccagagtgta aagtggtcaa atgtcgattt ccagtagtcg aaaatggaaa acagatatca 720

ggatttggaa aaaaatttta ctacaaagca acagttatgt ttgaatgcga taagggtttt 780

tacctcgatg gcagcgacac aattgtctgt gacagtaaca gtacttggga tcccccagtt 840

ccaaagtgtc ttaaaggtcc taggcctact tacaagcctc cagtctcaaa ttatccagga 900

tatcctaaac ctgaggaagg aatacttgac agtttggatg tttgggtcat tgctgtgatt 960

gttattgcca tagttgttgg agttgcagta atttgtgttg tcccgtacag atatcttcaa 1020

aggaggaaga agaaaggcac atacctaact gatgagaccc acagagaagt aaaatttact 1080

tctctctga 1089

<210> 6

<211> 1110

<212> DNA

<213> homo sapiens

<400> 6
atggagcctc ccggccgccg cgagtgtccc tttccttcct ggcgctttcc tgggttgctt 60

ctggcggcca tggtgttgct gctgtactcc ttctccgatg cctgtgagga gccaccaaca 120

tttgaagcta tggagctcat tggtaaacca aaaccctact atgagattgg tgaacgagta 180

gattataagt gtaaaaaagg atacttctat atacctcctc ttgccaccca tactatttgt 240

gatcggaatc atacatggct acctgtctca gatgacgcct gttatagaga aacatgtcca 300

tatatacggg atcctttaaa tggccaagca gtccctgcaa atgggactta cgagtttggt 360

tatcagatgc actttatttg taatgagggt tattacttaa ttggtgaaga aattctatat 420

tgtgaactta aaggatcagt agcaatttgg agcggtaagc ccccaatatg tgaaaaggtt 480

ttgtgtacac cacctccaaa aataaaaaat ggaaaacaca cctttagtga agtagaagta 540

tttgagtatc ttgatgcagt aacttatagt tgtgatcctg cacctggacc agatccattt 600

tcacttattg gagagagcac gatttattgt ggtgacaatt cagtgtggag tcgtgctgct 660

ccagagtgta aagtggtcaa atgtcgattt ccagtagtcg aaaatggaaa acagatatca 720

ggatttggaa aaaaatttta ctacaaagca acagttatgt ttgaatgcga taagggtttt 780

tacctcgatg gcagcgacac aattgtctgt gacagtaaca gtacttggga tcccccagtt 840

ccaaagtgtc ttaaaggtcc taggcctact tacaagcctc cagtctcaaa ttatccagga 900

tatcctaaac ctgaggaagg aatacttgac agtttggatg tttgggtcat tgctgtgatt 960

gttattgcca tagttgttgg agttgcagta atttgtgttg tcccgtacag atatcttcaa 1020

aggaggaaga agaaagggaa agcagatggt ggagctgaat atgccactta ccagactaaa 1080

tcaaccactc cagcagagca gagaggctga 1110

<210> 7

<211> 1110

<212> DNA

<213> homo sapiens

<400> 7
atggagcctc ccggccgccg cgagtgtccc tttccttcct ggcgctttcc tgggttgctt 60

ctggcggcca tggtgttgct gctgtactcc ttctccgatg cctgtgagga gccaccaaca 120

tttgaagcta tggagctcat tggtaaacca aaaccctact atgagattgg tgaacgagta 180

gattataagt gtaaaaaagg atacttctat atacctcctc ttgccaccca tactatttgt 240

gatcggaatc atacatggct acctgtctca gatgacgcct gttatagaga aacatgtcca 300

tatatacggg atcctttaaa tggccaagca gtccctgcaa atgggactta cgagtttggt 360

tatcagatgc actttatttg taatgagggt tattacttaa ttggtgaaga aattctatat 420

tgtgaactta aaggatcagt agcaatttgg agcggtaagc ccccaatatg tgaaaaggtt 480

ttgtgtacac cacctccaaa aataaaaaat ggaaaacaca cctttagtga agtagaagta 540

tttgagtatc ttgatgcagt aacttatagt tgtgatcctg cacctggacc agatccattt 600

tcacttattg gagagagcac gatttattgt ggtgacaatt cagtgtggag tcgtgctgct 660

ccagagtgta aagtggtcaa atgtcgattt ccagtagtcg aaaatggaaa acagatatca 720

ggatttggaa aaaaatttta ctacaaagca acagttatgt ttgaatgcga taagggtttt 780

tacctcgatg gcagcgacac aattgtctgt gacagtaaca gtacttggga tcccccagtt 840

ccaaagtgtc ttaaaggtcc taggcctact tacaagcctc cagtctcaaa ttatccagga 900

tatcctaaac ctgaggaagg aatacttgac agtttggatg tttgggtcat tgctgtgatt 960

gttattgcca tagttgttgg agttgcagta atttgtgttg tcccgtacag atatcttcaa 1020

aggaggaaga agaaagggaa agcagatggt ggagctgaat atgccactta ccagactaaa 1080

tcaaccactc cagcagagca gagaggctga 1110

<210> 8

<211> 1134

<212> DNA

<213> homo sapiens

<400> 8

atggagcctc ccggccgccg cgagtgtccc tttccttcct ggcgctttcc tgggttgctt 60

ctggcggcca tggtgttgct gctgtactcc ttctccgatg cctgtgagga gccaccaaca 120

tttgaagcta tggagctcat tggtaaacca aaaccctact atgagattgg tgaacgagta 180

gattataagt gtaaaaaagg atacttctat atacctcctc ttgccaccca tactatttgt 240

gatcggaatc atacatggct acctgtctca gatgacgcct gttatagaga aacatgtcca 300

tatatacggg atcctttaaa tggccaagca gtccctgcaa atgggactta cgagtttggt 360

tatcagatgc actttatttg taatgagggt tattacttaa ttggtgaaga aattctatat 420

tgtgaactta aaggatcagt agcaatttgg agcggtaagc ccccaatatg tgaaaaggtt 480

ttgtgtacac cacctccaaa aataaaaaat ggaaaacaca cctttagtga agtagaagta 540

tttgagtatc ttgatgcagt aacttatagt tgtgatcctg cacctggacc agatccattt 600

tcacttattg gagagagcac gatttattgt ggtgacaatt cagtgtggag tcgtgctgct 660

ccagagtgta aagtggtcaa atgtcgattt ccagtagtcg aaaatggaaa acagatatca 720

ggatttggaa aaaaatttta ctacaaagca acagttatgt ttgaatgcga taagggtttt 780

tacctcgatg gcagcgacac aattgtctgt gacagtaaca gtacttggga tcccccagtt 840

ccaaagtgtc ttaaagtgtc gacttcttcc actacaaaat ctccagcgtc cagtgcctca 900

ggtcctaggc ctacttacaa gcctccagtc tcaaattatc caggatatcc taaacctgag 960

gaaggaatac ttgacagttt ggatgtttgg gtcattgctg tgattgttat tgccatagtt 1020

gttggagttg cagtaatttg tgttgtcccg tacagatatc ttcaaaggag gaagaagaaa 1080

ggcacatacc taactgatga gacccacaga gaagtaaaat ttacttctct ctga 1134

<210> 9

<211> 1092

<212> DNA

<213> homo sapiens

<400> 9
atggagcctc ccggccgccg cgagtgtccc tttccttcct ggcgctttcc tgggttgctt 60

ctggcggcca tggtgttgct gctgtactcc ttctccgatg cctgtgagga gccaccaaca 120

tttgaagcta tggagctcat tggtaaacca aaaccctact atgagattgg tgaacgagta 180

gattataagt gtaaaaaagg atacttctat atacctcctc ttgccaccca tactatttgt 240

gatcggaatc atacatggct acctgtctca gatgacgcct gttatagaga aacatgtcca 300

tatatacggg atcctttaaa tggccaagca gtccctgcaa atgggactta cgagtttggt 360

tatcagatgc actttatttg taatgagggt tattacttaa ttggtgaaga aattctatat 420

tgtgaactta aaggatcagt agcaatttgg agcggtaagc ccccaatatg tgaaaaggtt 480

ttgtgtacac cacctccaaa aataaaaaat ggagaacaca cctttagtga agtagaagta 540

tttgagtatc ttgatgcagt aacttatagt tgtgatcctg cacctggacc agatccattt 600

tcacttattg gagagagcac gatttattgt ggtgacaatt cagtgtggag tcgtgctgct 660

ccagagtgta aagtggtcaa atgtcgattt ccagtagtcg aaaatggaaa acagatatca 720

ggatttggaa aaaaatttta ctacaaagca acagttatgt ttgaatgcga taagggtttt 780

tacctcgatg gcagcgacac aattgtctgt gacagtaaca gtacttggga tcccccagtt 840

ccaaagtgtc ttaaagtgtc gacttcttcc actacaaaat ctccagcgtc cagtgcctca 900

ggatatccta aacctgagga aggaatactt gacagtttgg atgtttgggt cattgctgtg 960

attgttattg ccatagttgt tggagttgca gtaatttgtg ttgtcccgta cagatatctt 1020

caaaggagga agaagaaagg cacataccta actgatgaga cccacagaga agtaaaattt 1080

acttctctct ga 1092

<210> 10

<211> 1095

<212> DNA

<213> homo sapiens

<400> 10
atggagcctc ccggccgccg cgagtgtccc tttccttcct ggcgctttcc tgggttgctt 60

ctggcggcca tggtgttgct gctgtactcc ttctccgatg cctgtgagga gccaccaaca 120

tttgaagcta tggagctcat tggtaaacca aaaccctact atgagattgg tgaacgagta 180

gattataagt gtaaaaaagg atacttctat atacctcctc ttgccaccca tactatttgt 240

gatcggaatc atacatggct acctgtctca gatgacgcct gttatagaga aacatgtcca 300

tatatacggg atcctttaaa tggccaagca gtccctgcaa atgggactta cgagtttggt 360

tatcagatgc actttatttg taatgagggt tattacttaa ttggtgaaga aattctatat 420

tgtgaactta aaggatcagt agcaatttgg agcggtaagc ccccaatatg tgaaaaggtt 480

ttgtgtacac cacctccaaa aataaaaaat ggaaaacaca cctttagtga agtagaagta 540

tttgagtatc ttgatgcagt aacttatagt tgtgatcctg cacctggacc agatccattt 600

tcacttattg gagagagcac gatttattgt ggtgacaatt cagtgtggag tcgtgctgct 660

ccagagtgta aagtggtcaa atgtcgattt ccagtagtcg aaaatggaaa acagatatca 720

ggatttggaa aaaaatttta ctacaaagca acagttatgt ttgaatgcga taagggtttt 780

tacctcgatg gcagcgacac aattgtctgt gacagtaaca gtacttggga tcccccagtt 840

ccaaagtgtc ttaaaggtcc taggcctact tacaagcctc cagtctcaaa ttatccagga 900

tatcctaaac ctgaggaagg aatacttgac agtttggatg tttgggtcat tgctgtgatt 960

gttattgcca tagttgttgg agttgcagta atttgtgttg tcccgtacag atatcttcaa 1020

aggaaagcag atggtggagc tgaatatgcc acttaccaga ctaaatcaac cactccagca 1080

gagcagagag gctga 1095

<210> 11

<211> 1134

<212> DNA

<213> homo sapiens

<220>

<221> misc_feature

<222> (35)..(35)

<223> n can be A, C, G or T

<220>

<221> misc_feature

<222> (1109)..(1109)

<223> n can be A, C, G or T

<400> 11
atggagcctc ccggccgccg cgagtgtccc tttcnttcct ggcgctttcc tgggttgctt 60

ctggcggcca tggtgttgct gctgtactcc ttctccgatg cctgtgagga gccaccaaca 120

tttgaagcta tggagctcat tggtaaacca aaaccctact atgggattgg tgaacgagta 180

gattataagt gtaaaaaagg atacttctat atacctcctc ttgccaccca tactatttgt 240

gatcggaatc atacatggct acctgtctca gatgacgcct gttatagaga aacatgtcca 300

tatatacggg atcctttaaa tggccaagca gtccctgcaa atgggactta cgagtttggt 360

tatcagatgc actttatttg taatgagggt tattacttaa ttggtgaaga aattctatat 420

tgtgaactta aaggatcagt agcaatttgg agcggtaagc ccccaatatg tgaaaaggtt 480

ttgtgtacac cacctccaaa aataaaaaat ggaaaacaca cctttagtga agtagaagta 540

tttgagtatc ttgatgcagt aacttatagt tgtgatcctg cacctggacc agatccattt 600

tcacttattg gagagagcac gatttattgt ggtgacaatt cagtgtggag tcgtgctgct 660

ccagagtgta aagtggtcaa atgtcgattt ccagtagtcg aaaatggaaa acagatatca 720

ggatttggaa aaaaatttta ctacaaagca acagttatgt ttgaatgcga taagggtttt 780

tacctcgatg gcagcgacac aattgtctgt gacagtaaca gtacttggga tcccccagtt 840

ccaaagtgtc ttaaagtgtc gacttcttcc actacaaaat ctccagcgtc cagtgcctca 900

ggtcctaggt ctacttacaa gcctccagtc tcaaattatc caggatatcc taaacctgag 960

gaaggaatac ttgacagttt ggatgtttgg gtcattgctg tgattgttat tgccatagtt 1020

gttggagttg cagtaatttg tgttgtcccg tacagatatc ttcaaaggag gaagaagaaa 1080

ggcacatacc taactgatga gacccacana gaagtaaaat ttacttctct ctga 1134

<210> 12

<211> 942

<212> DNA

<213> homo sapiens

<400> 12
atggagcctc ccggccgccg cgagtgtccc tttccttcct ggcgctttcc tgggttgctt 60

ctggcggcca tggtgttgct gctgtactcc ttctccgatg cctgtgagga gccaccaaca 120

tttgaagcta tggagctcat tggtaaacca aaaccctact atgagattgg tgaacgagta 180

gattataagt gtaaaaaagg atacttctat atacctcctc ttgccaccca tactatttgt 240

gatcggaatc atacatggct acctgtctca gatgacgcct gttatagaga aacatgtcca 300

tatatacggg atcctttaaa tggccaagca gtccctgcaa atgggactta cgagtttggt 360

tatcagatgc actttatttg taatgagggt tattacttaa ttggtgaaga aattctatat 420

tgtgaactta aaggatcagt agcaatttgg agcggtaagc ccccaatatg tgaaaaggtt 480

ttgtgtacac cacctccaaa aataaaaaat ggaaaacaca cctttagtga agtagaagta 540

tttgagtatc ttgatgcagt aacttatagt tgtgatcctg cacctggacc agatccattt 600

tcacttattg gagagagcac gatttattgt ggtgacaatt cagtgtggag tcgtgctgct 660

ccagagtgta aagtggtcaa atgtcgattt ccagtagtcg aaaatggaaa acagatatca 720

ggatttggaa aaaaatttta ctacaaagca acagttatgt ttgaatgcga taagggtttt 780

tacctcgatg gcagcgacac aattgtctgt gacagtaaca gtacttggga tcccccagtt 840

ccaaagtgtc ttaaaggtcc taggcctact tacaagcctc cagtctcaaa ttatccagga 900

tatcctaaac ctgaggaagg aatacttgac agtttggatg tt 942

<210>  13

<211>  314

<212>  PRT

<213>  homo sapiens

<400>  13

Met Glu Pro Pro Gly Arg Arg Glu Cys Pro Phe Pro Ser Trp Arg Phe
1               5                   10                  15

Pro Gly Leu Leu Leu Ala Ala Met Val Leu Leu Leu Tyr Ser Phe Ser
            20                  25                  30

Asp Ala Cys Glu Glu Pro Pro Thr Phe Glu Ala Met Glu Leu Ile Gly
        35                  40                  45

Lys Pro Lys Pro Tyr Tyr Glu Ile Gly Glu Arg Val Asp Tyr Lys Cys
    50                  55                  60

Lys Lys Gly Tyr Phe Tyr Ile Pro Pro Leu Ala Thr His Thr Ile Cys
65                  70                  75                  80

Asp Arg Asn His Thr Trp Leu Pro Val Ser Asp Asp Ala Cys Tyr Arg
                85                  90                  95

Glu Thr Cys Pro Tyr Ile Arg Asp Pro Leu Asn Gly Gln Ala Val Pro
            100                 105                 110

Ala Asn Gly Thr Tyr Glu Phe Gly Tyr Gln Met His Phe Ile Cys Asn
        115                 120                 125

Glu Gly Tyr Tyr Leu Ile Gly Glu Glu Ile Leu Tyr Cys Glu Leu Lys
    130                 135                 140

Gly Ser Val Ala Ile Trp Ser Gly Lys Pro Pro Ile Cys Glu Lys Val
145                 150                 155                 160

Leu Cys Thr Pro Pro Pro Lys Ile Lys Asn Gly Lys His Thr Phe Ser
                165                 170                 175

Glu Val Glu Val Phe Glu Tyr Leu Asp Ala Val Thr Tyr Ser Cys Asp
            180                 185                 190

Pro Ala Pro Gly Pro Asp Pro Phe Ser Leu Ile Gly Glu Ser Thr Ile
        195                 200                 205

Tyr Cys Gly Asp Asn Ser Val Trp Ser Arg Ala Ala Pro Glu Cys Lys
    210                 215                 220

Val Val Lys Cys Arg Phe Pro Val Val Glu Asn Gly Lys Gln Ile Ser
225                 230                 235                 240

Gly Phe Gly Lys Lys Phe Tyr Tyr Lys Ala Thr Val Met Phe Glu Cys
                245                 250                 255

Asp Lys Gly Phe Tyr Leu Asp Gly Ser Asp Thr Ile Val Cys Asp Ser
            260                 265                 270

Asn Ser Thr Trp Asp Pro Pro Val Pro Lys Cys Leu Lys Gly Pro Arg
        275                 280                 285

Pro Thr Tyr Lys Pro Pro Val Ser Asn Tyr Pro Gly Tyr Pro Lys Pro
    290                 295                 300

Glu Glu Gly Ile Leu Asp Ser Leu Asp Val
305                 310

<210>  14

<211>  945

<212>  DNA

<213>  homo sapiens


<400>  14
atggagcctc ccggccgccg cgagtgtccc tttccttcct ggcgctttcc tgggttgctt      60

ctggcggcca tggtgttgct gctgtactcc ttctccgatg cctgtgagga gccaccaaca     120

tttgaagcta tggagctcat tggtaaacca aaaccctact atgagattgg tgaacgagta     180

gattataagt gtaaaaaagg atacttctat atacctcctc ttgccaccca tactatttgt     240

gatcggaatc atacatggct acctgtctca gatgacgcct gttatagaga aacatgtcca     300

tatatacggg atcctttaaa tggccaagca gtccctgcaa atgggactta cgagtttggt     360

tatcagatgc actttatttg taatgagggt tattacttaa ttggtgaaga aattctatat     420

tgtgaactta aaggatcagt agcaatttgg agcggtaagc ccccaatatg tgaaaaggtt     480

ttgtgtacac cacctccaaa aataaaaaat ggagaacaca cctttagtga agtagaagta     540

tttgagtatc ttgatgcagt aacttatagt tgtgatcctg cacctggacc agatccattt     600

tcacttattg gagagagcac gatttattgt ggtgacaatt cagtgtggag tcgtgctgct     660

ccagagtgta aagtggtcaa atgtcgattt ccagtagtcg aaaatggaaa acagatatca 720

ggatttggaa aaaaatttta ctacaaagca acagttatgt ttgaatgcga taagggtttt 780

tacctcgatg gcagcgacac aattgtctgt gacagtaaca gtacttggga tcccccagtt 840

ccaaagtgtc ttaaagtgtc gacttcttcc actacaaaat ctccagcgtc cagtgcctca 900

ggatatccta aacctgagga aggaatactt gacagtttgg atgtt 945

<210> 15

<211> 315

<212> PRT

<213> homo sapiens

<400> 15

Met Glu Pro Pro Gly Arg Arg Glu Cys Pro Phe Pro Ser Trp Arg Phe
1               5                   10                  15

Pro Gly Leu Leu Leu Ala Ala Met Val Leu Leu Leu Tyr Ser Phe Ser
            20                  25                  30

Asp Ala Cys Glu Glu Pro Pro Thr Phe Glu Ala Met Glu Leu Ile Gly
        35                  40                  45

Lys Pro Lys Pro Tyr Tyr Glu Ile Gly Glu Arg Val Asp Tyr Lys Cys
    50                  55                  60

Lys Lys Gly Tyr Phe Tyr Ile Pro Pro Leu Ala Thr His Thr Ile Cys
65                  70                  75                  80

Asp Arg Asn His Thr Trp Leu Pro Val Ser Asp Asp Ala Cys Tyr Arg
                85                  90                  95

Glu Thr Cys Pro Tyr Ile Arg Asp Pro Leu Asn Gly Gln Ala Val Pro
            100                 105                 110

Ala Asn Gly Thr Tyr Glu Phe Gly Tyr Gln Met His Phe Ile Cys Asn
        115                 120                 125

Glu Gly Tyr Tyr Leu Ile Gly Glu Glu Ile Leu Tyr Cys Glu Leu Lys
    130                 135                 140

Gly Ser Val Ala Ile Trp Ser Gly Lys Pro Pro Ile Cys Glu Lys Val
145                 150                 155                 160

Leu Cys Thr Pro Pro Pro Lys Ile Lys Asn Gly Glu His Thr Phe Ser
                165                 170                 175

Glu Val Glu Val Phe Glu Tyr Leu Asp Ala Val Thr Tyr Ser Cys Asp
            180                 185                 190

Pro Ala Pro Gly Pro Asp Pro Phe Ser Leu Ile Gly Glu Ser Thr Ile
        195                 200                 205

Tyr Cys Gly Asp Asn Ser Val Trp Ser Arg Ala Ala Pro Glu Cys Lys
    210                 215                 220

Val Val Lys Cys Arg Phe Pro Val Val Glu Asn Gly Lys Gln Ile Ser
225                 230                 235                 240

Gly Phe Gly Lys Lys Phe Tyr Tyr Lys Ala Thr Val Met Phe Glu Cys
                245                 250                 255

Asp Lys Gly Phe Tyr Leu Asp Gly Ser Asp Thr Ile Val Cys Asp Ser
            260                 265                 270

Asn Ser Thr Trp Asp Pro Pro Val Pro Lys Cys Leu Lys Val Ser Thr
        275                 280                 285

Ser Ser Thr Thr Lys Ser Pro Ala Ser Ser Ala Ser Gly Tyr Pro Lys
    290                 295                 300

Pro Glu Glu Gly Ile Leu Asp Ser Leu Asp Val
305                 310                 315

<210>  16

<211>  987

<212>  DNA

<213>  homo sapiens

<400>  16
atggagcctc ccggccgccg cgagtgtccc tttccttcct ggcgctttcc tgggttgctt      60

ctggcggcca tggtgttgct gctgtactcc ttctccgatg cctgtgagga gccaccaaca     120

tttgaagcta tggagctcat tggtaaacca aaaccctact atgagattgg tgaacgagta     180

gattataagt gtaaaaaagg atacttctat atacctcctc ttgccaccca tactatttgt     240

gatcggaatc atacatggct acctgtctca gatgacgcct gttatagaga aacatgtcca     300

tatatacggg atcctttaaa tggccaagca gtccctgcaa atgggactta cgagtttggt     360

tatcagatgc actttatttg taatgagggt tattacttaa ttggtgaaga aattctatat 420

tgtgaactta aaggatcagt agcaatttgg agcggtaagc ccccaatatg tgaaaaggtt 480

ttgtgtacac cacctccaaa aataaaaaat ggaaaacaca cctttagtga agtagaagta 540

tttgagtatc ttgatgcagt aacttatagt tgtgatcctg cacctggacc agatccattt 600

tcacttattg gagagagcac gatttattgt ggtgacaatt cagtgtggag tcgtgctgct 660

ccagagtgta aagtggtcaa atgtcgattt ccagtagtcg aaaatggaaa acagatatca 720

ggatttggaa aaaaatttta ctacaaagca acagttatgt ttgaatgcga taagggtttt 780

tacctcgatg gcagcgacac aattgtctgt gacagtaaca gtacttggga tcccccagtt 840

ccaaagtgtc ttaaagtgtc gacttcttcc actacaaaat ctccagcgtc cagtgcctca 900

ggtcctaggc ctacttacaa gcctccagtc tcaaattatc caggatatcc taaacctgag 960

gaaggaatac ttgacagttt ggatgtt 987

<210> 17

<211> 329

<212> PRT

<213> homo sapiens

<400> 17

Met Glu Pro Pro Gly Arg Arg Glu Cys Pro Phe Pro Ser Trp Arg Phe
1               5                   10                  15

Pro Gly Leu Leu Leu Ala Ala Met Val Leu Leu Leu Tyr Ser Phe Ser
            20                  25                  30

Asp Ala Cys Glu Glu Pro Pro Thr Phe Glu Ala Met Glu Leu Ile Gly
        35                  40                  45

Lys Pro Lys Pro Tyr Tyr Glu Ile Gly Glu Arg Val Asp Tyr Lys Cys
    50                  55                  60

Lys Lys Gly Tyr Phe Tyr Ile Pro Pro Leu Ala Thr His Thr Ile Cys
65                  70                  75                  80

Asp Arg Asn His Thr Trp Leu Pro Val Ser Asp Asp Ala Cys Tyr Arg
                85                  90                  95

Glu Thr Cys Pro Tyr Ile Arg Asp Pro Leu Asn Gly Gln Ala Val Pro
            100                 105                 110

Ala Asn Gly Thr Tyr Glu Phe Gly Tyr Gln Met His Phe Ile Cys Asn
        115                 120                 125

Glu Gly Tyr Tyr Leu Ile Gly Glu Glu Ile Leu Tyr Cys Glu Leu Lys
    130                 135                 140

Gly Ser Val Ala Ile Trp Ser Gly Lys Pro Pro Ile Cys Glu Lys Val
145                 150                 155                 160

Leu Cys Thr Pro Pro Pro Lys Ile Lys Asn Gly Lys His Thr Phe Ser
                165                 170                 175

Glu Val Glu Val Phe Glu Tyr Leu Asp Ala Val Thr Tyr Ser Cys Asp
            180                 185                 190

Pro Ala Pro Gly Pro Asp Pro Phe Ser Leu Ile Gly Glu Ser Thr Ile
        195                 200                 205

Tyr Cys Gly Asp Asn Ser Val Trp Ser Arg Ala Ala Pro Glu Cys Lys
    210                 215                 220

Val Val Lys Cys Arg Phe Pro Val Val Glu Asn Gly Lys Gln Ile Ser
225                 230                 235                 240

Gly Phe Gly Lys Lys Phe Tyr Tyr Lys Ala Thr Val Met Phe Glu Cys
                245                 250                 255

Asp Lys Gly Phe Tyr Leu Asp Gly Ser Asp Thr Ile Val Cys Asp Ser
            260                 265                 270

Asn Ser Thr Trp Asp Pro Pro Val Pro Lys Cys Leu Lys Val Ser Thr
        275                 280                 285

Ser Ser Thr Thr Lys Ser Pro Ala Ser Ser Ala Ser Gly Pro Arg Pro
    290                 295                 300

Thr Tyr Lys Pro Pro Val Ser Asn Tyr Pro Gly Tyr Pro Lys Pro Glu
305                 310                 315                 320

Glu Gly Ile Leu Asp Ser Leu Asp Val
                325

<210>  18

<211>  1671

<212>  DNA

<213>  artificial sequence

<220>

<223>  Construct encoding a fusion polypeptide between the extracellular
       domain of CD46 at the N-terminus and a human Fc-gamma3 domain at
       the C-terminus

<400>  18
atggagcctc ccggccgccg cgagtgtccc tttccttcct ggcgctttcc tgggttgctt      60

ctggcggcca tggtgttgct gctgtactcc ttctccgatg cctgtgagga gccaccaaca     120

tttgaagcta tggagctcat tggtaaacca aaaccctact atgagattgg tgaacgagta     180

gattataagt gtaaaaaagg atacttctat atacctcctc ttgccaccca tactatttgt     240

gatcggaatc atacatggct acctgtctca gatgacgcct gttatagaga aacatgtcca     300

tatatacggg atcctttaaa tggccaagca gtccctgcaa atgggactta cgagtttggt     360

tatcagatgc actttatttg taatgagggt tattacttaa ttggtgaaga aattctatat     420

tgtgaactta aaggatcagt agcaatttgg agcggtaagc ccccaatatg tgaaaaggtt     480

ttgtgtacac cacctccaaa aataaaaaat ggaaaacaca cctttagtga agtagaagta     540

tttgagtatc ttgatgcagt aacttatagt tgtgatcctg cacctggacc agatccattt     600

tcacttattg gagagagcac gatttattgt ggtgacaatt cagtgtggag tcgtgctgct     660

ccagagtgta aagtggtcaa atgtcgattt ccagtagtcg aaaatggaaa acagatatca     720

ggatttggaa aaaaatttta ctacaaagca acagttatgt ttgaatgcga taagggtttt     780

tacctcgatg gcagcgacac aattgtctgt gacagtaaca gtacttggga tcccccagtt     840

ccaaagtgtc ttaaagtgtc gacttcttcc actacaaaat ctccagcgtc cagtgcctca     900

ggtcctaggc ctacttacaa gcctccagtc tcaaattatc caggatatcc taaacctgag     960

gaaggaatac ttgacagttt ggatgttaag cttactcaca catgcccacc gtgcccagca    1020

cctgaagccg agggggcacc gtcagtcttc ctcttccccc caaaacccaa ggacaccctc    1080

atgatctccc ggacccctga ggtcacatgc gtggtggtgg acgtgagcca cgaagaccct    1140

gaggtcaagt tcaactggta cgtggacggc gtggaggtgc ataatgccaa gacaaagccg    1200

cgggaggagc agtacaacag cacgtaccgt gtggtcagcg tcctcaccgt cctgcaccag    1260

gactggctga atggcaagga gtacaagtgc aaggtctcca acaaagccct cccagcctcc    1320

atcgagaaaa ccatctccaa agccaaaggg cagccccgag aaccacaggt gtacaccctg    1380

cccccatccc gggatgagct gaccaagaac caggtcagcc tgacctgcct ggtcaaaggc    1440

ttctatccca gcgacatcgc cgtggagtgg gagagcaatg ggcagccgga gaacaactac    1500

aagaccacgc ctcccgtgtt ggactccgac ggctccttct tcctctacag caagctcacc    1560

gtggacaaga gcaggtggca gcaggggaac gtcttctcat gctccgtgat gcatgaggct    1620

ctgcacaacc actacacgca gaagagcctc tccctgtctc cgggtaaatg a             1671

<210>  19

<211>  556

<212>  PRT

<213>  artificial sequence

<220>

<223>  fusion polypeptide between the extracellular domain of CD46 at th
       e N-terminus and a human Fc-gamma3 domain at the C-terminus

<400>  19

<210>  20

<211>  1746

<212>  DNA

<213>  artificial sequence

<220>

<223>  Construct encoding a fusion polypeptide between the extracellular
        domain of CD46 at the N-terminus and the scFv FRP5 against ErbB-
       2 at the C-terminus

<400>  20
atggagcctc ccggccgccg cgagtgtccc tttccttcct ggcgctttcc tgggttgctt      60

ctggcggcca tggtgttgct gctgtactcc ttctccgatg cctgtgagga gccaccaaca     120

tttgaagcta tggagctcat tggtaaacca aaaccctact atgagattgg tgaacgagta     180

gattataagt gtaaaaaagg atacttctat atacctcctc ttgccaccca tactatttgt     240

gatcggaatc atacatggct acctgtctca gatgacgcct gttatagaga aacatgtcca     300

tatatacggg atcctttaaa tggccaagca gtccctgcaa atgggactta cgagtttggt 360

tatcagatgc actttatttg taatgagggt tattacttaa ttggtgaaga aattctatat 420

tgtgaactta aaggatcagt agcaatttgg agcggtaagc ccccaatatg tgaaaaggtt 480

ttgtgtacac cacctccaaa aataaaaaat ggaaaacaca cctttagtga agtagaagta 540

tttgagtatc ttgatgcagt aacttatagt tgtgatcctg cacctggacc agatccattt 600

tcacttattg gagagagcac gatttattgt ggtgacaatt cagtgtggag tcgtgctgct 660

ccagagtgta aagtggtcaa atgtcgattt ccagtagtcg aaaatggaaa acagatatca 720

ggatttggaa aaaaatttta ctacaaagca acagttatgt ttgaatgcga taagggtttt 780

tacctcgatg gcagcgacac aattgtctgt gacagtaaca gtacttggga tcccccagtt 840

ccaaagtgtc ttaaagtgtc gacttcttcc actacaaaat ctccagcgtc cagtgcctca 900

ggtcctaggc ctacttacaa gcctccagtc tcaaattatc caggatatcc taaacctgag 960

gaaggaatac ttgacagttt ggatgttggc ggccgctctc aggtacaact gcagcagtct 1020

ggacctgaac tgaagaagcc tggagagaca gtcaagatct cctgcaaggc ctctgggtat 1080

cctttcacaa actatggaat gaactgggtg aagcaggctc caggacaggg tttaaagtgg 1140

atgggctgga ttaacacttc cactggagag tcaacatttg ctgatgactt caagggacgg 1200

tttgacttct ctttggaaac ctctgccaac actgcctatt tgcagatcaa caacctcaaa 1260

agtgaagaca tggctacata tttctgtgca agatgggagg tttaccacgg ctacgttcct 1320

tactggggcc aagggaccac ggtcaccgtt tcctctggcg gtggcggttc tggtggcggt 1380

ggctccggcg gtggcggttc tgacatccag ctgacccagt ctcacaaatt cctgtccact 1440

tcagtaggag acagggtcag catcacctgc aaggccagtc aggatgtgta taatgctgtt 1500

gcctggtatc aacagaaacc aggacaatct cctaaacttc tgatttactc ggcatcctcc 1560

cggtacactg gagtcccttc tcgcttcact ggcagtggct ctgggccgga tttcactttc 1620

accatcagca gtgtgcaggc tgaagacctg gcagtttatt tctgtcagca acattttcgt 1680

actccattca cgttcggctc ggggacaaaa ttggagatcg actacaagga tgacgacgac 1740

aagtag 1746

<210> 21

<211> 581

<212> PRT

<213> artificial sequence

<220>

<223>  fusion polypeptide between the extracellular domain of CD46 at th
       e N-terminus and the scFv FRP5 against ErbB-2 at the C-terminus

<400>  21